# EUROPEAN PATENT APPLICATION

(11) **EP 4 571 315 A1**
(43) Date of publication of application: **18.06.2025**
(21) Application number: 23852041.5
(22) Date of filing: 26.09.2023
(51) Int. Cl.: G01N 33/68

(54) **DIAGNOSTIC USE OF HIGHLY TOXIC AMYLOID OLIGOMER**

(30) Priority: 09.08.2022 CN 202210951282
(71) Applicant: Shen Zhen Wisdom Biopharm Co., Ltd., Shenzhen, Guangdong 518067 (CN)
(72) Inventor: LIU, Ruitian, Shenzhen, Guangdong 518067 (CN); HUANG, Yaru, Shenzhen, Guangdong 518067 (CN); XIE, Xixiu, Shenzhen, Guangdong 518067 (CN); YU, Xiaolin, Shenzhen, Guangdong 518067 (CN); JI, Shaoyang, Shenzhen, Guangdong 518067 (CN)
(74) Representative: Høiberg P/S
(86) International application number: PCT/CN2023/121351
(87) International publication number: WO 2024/032823

(57) **Abstract**

A use of a new highly toxic amyloid oligomer Apo*3F as a target for diagnosing Alzheimer's disease (AD) in the early stage and the middle-late stage and mild cognitive impairment (MCI) caused by AD. The Aβo*3F specifically binds to an antibody 3F and is present in cerebrospinal fluid (CSF), blood and/or brain tissue of AD patients and patients with MCI caused by AD, and the levels show highly significant differences in CSF, blood and/or brain tissue of AD patients, MCI patients and healthy elderly persons. In addition, the Apo*3F is an ultra-highly toxic oligomer, is the most important toxic component in an Aβ oligomer mixture, has a strong pathogenic effect, and plays a key role in the occurrence and development of AD.

## Description

### Technical Field

The present invention relates to the diagnostic use of highly toxic amyloid oligomers. Specifically, the present invention relates to the use of novel highly toxic amyloid oligomer Apo*3F as a target for diagnosing early and mid-late stage Alzheimer's disease (AD) and AD-derived mild cognitive impairment (MCI).

### Background

Alzheimer's disease (AD), which is also called senile dementia, is a chronic neurodegenerative disease. There are about 50 million AD patients worldwide. So far, there is no specific effective therapeutic drug or means, which has brought a heavy burden to mankind. AD has a long development course. At first, patients show no substantial clinical symptoms, and gradually develop memory loss and personality and behavior changes. In the later stage, the neurons in AD patients' brain die extensively, the brain shrinks significantly, and the therapeutic drugs are difficult to take effect. Clinical trials in recent years have shown that early intervention for AD patients with early detection and early warning can delay the development of AD. In order to achieve early treatment and intervention of AD patients, they should be identified and diagnosed in a timely and accurate manner first, however, there is currently no ideal early diagnosis method and technology for clinical application. Studies have shown that changes in amyloid β (Aβ) protein that induce the development of AD have already appeared 15 to 20 years before the onset of clinical symptoms.

The pathological characteristics of AD are senile plaques formed by Aβ aggregation and neurofibrillary tangles formed by tau protein aggregation in the brain. Aβ can aggregate into oligomers, pre-fibrils and mature fibers. Aβ oligomers are the most neurotoxic aggregates, and the most reported forms are Aβ dimers, trimers, ADDLs, etc. Aβ oligomers can be divided into two types according to their molecular weight, low molecular weight and high molecular weight, and high molecular weight oligomers have greater neurotoxicity. Aβ dimers are the smallest Aβ oligomers, and it is generally believed that Aβ dimers are the basic building blocks of Aβ oligomers. Aβ dimers show an elevated level in the brains of AD patients and AD transgenic mice, and are stable in SDS and strong denaturants, with certain neurotoxicity. In addition to Aβ dimers, Aβ trimers are also considered to be the aggregation units of various Aβ oligomers such as hexamers and dodecamers. Aβ trimers appear early in the brains of AD patients and AD transgenic mice, but there is no significant correlation between Aβ trimers and Aβ plaque deposition, and their toxicity is still controversial. In addition, Aβ oligomers can also form spherical oligomers (ASPD) with a diameter of about 12 nm and diffusible oligomers (ADDL) with a diameter of 5-6 nm after further aggregation. Both of these polymer forms are considered to be Aβ oligomers with unique conformation and neurotoxicity. At present, although a variety of Aβ oligomer forms have been discovered, it is still unknown which Aβ oligomers are the most toxic ones and play a major role in the occurrence and development of AD. Moreover, the methods for specifically detecting oligomers, especially toxic oligomers, are very limited, and it is particularly difficult to obtain antibodies that specifically bind to toxic amyloid oligomers, which seriously limits the clinical detection and development of therapeutic agents for Aβ oligomers.

A large number of studies have shown that the severity of neurodegenerative diseases is closely related to the level of amyloid oligomers in the patient's brain. Aβ oligomers play a key role in the occurrence and development of AD by causing functional neuronal death, cognitive impairment and dementia. However, there are many types of Aβ oligomers, which can affect the function of central nervous system through various mechanisms. The toxicity of various Aβ oligomers differ from each other, and there are also great differences in Aβ oligomer's size, conformation, aggregation mode, toxicity and timing of appearance in the brain. Although many studies have shown that some Aβ oligomers, such as dimers, trimers, ADDL, etc., can play a neurotoxic role, our current understanding on the Aβ oligomer form that really plays a key pathogenic role is not deep enough. Therefore, determining the key toxic oligomers that are closely related to the occurrence and development of AD can provide ideal markers for early warning and early diagnosis of AD, and can also provide ideal targets for the treatment of AD.

In addition to imaging tests, specific biomarkers are also the basis for clinical diagnosis and detection of AD. At present, the commonly recognized AD diagnostic markers in the industry are Aβ42, Aβ40, total tau protein (T-Tau) and phosphorylated tau protein (P-Tau) in cerebrospinal fluid (CSF). Other markers have also been reported, such as sAPPα, sAPPβ, BACE1, Aβ oligomers, total Aβ, axon and synapse markers, etc. These CSF markers show high diagnostic accuracy. Combined with other diagnostic indicators of AD, the diagnostic sensitivity and specificity of AD can reach 85-90%. Compared with CSF, blood is easy to obtain and less invasive, making it an ideal sample source for clinical testing. At present, many studies have begun to distinguish AD patients from normal people based on the content of peripheral blood proteins, lipids and metabolites, etc., such as the content of Aβ42 dimers on peripheral blood cell membranes, the content of plasma gelsolin (GSN) that depolymerizes Aβ42 fibers, and the content of MMP3, the main degrading enzyme of GSN, etc. Recently, the detection of specific components in peripheral blood such as Tau181, Tau217, and Aβ content has also become a hotspot.

The main AD biochemical detection methods focus on the determination of Aβ and Tau protein markers in CSF. A large number of studies have shown that in the process of normal people developing into early cognitive impairment (MCI) and AD patients, the level of Aβ42 monomer in CSF gradually decreases, the level of Aβ42 oligomer and T-Tau gradually increases, and the ratio of P-Tau to Aβ42 changes more significantly. However, obtaining CSF samples requires lumbar puncture, which is very invasive and it is difficult for MCI patients and mild AD patients to accept it. In addition, due to the lack of uniformity in CSF sample pretreatment and detection methods, the test results from different manufacturers and different laboratories vary greatly, which seriously limits the clinical promotion and application of this method. Compared with CSF, blood-based testing requires lower costs and has higher compliance. However, the markers that can be used for AD diagnosis has low contents and limited types in blood, and they are greatly affected by peripheral tissues and organs, which hinders the development of AD diagnostic methods. At present, the most advanced method around the world is to detect the ratio of Aβ42 to Aβ 40 in the blood, as well as the content of proteins such as P-Tau181 and P-Tau217, by liquid chromatography-mass spectrometry (LC-MS) and single molecule immunoassay (SIMOA). Although these indicators in the blood have some diagnostic significance, their specificity is poor, and a single indicator cannot distinguish the target population well. The reason is that the detection of Aβ protein or phosphorylated Tau protein is detecting the total amount of these proteins, including their monomers and various aggregates with different toxicities. Monomers and some types of oligomers have low cytotoxicity, but they have high content and made great contribution to the overall level of Aβ, which seriously interferes with the detection of amyloid oligomers having high toxicity and key pathogenic effects. Only the oligomers with high toxicity are directly related to the occurrence and development of AD. Its appearance and level are directly and accurately related to the pathological changes in the patient's brain. Specific detection on these oligomers is more valuable and meaningful.

Therefore, there is an urgent need for a highly sensitive and specific detection of specific Aβ oligomers' content in the blood in order to diagnose AD.

### Summary of the invention

One aspect of the present invention relates to a method for diagnosing whether a subject suffers from early and mid-late stage Alzheimer's disease (AD) or AD-derived mild cognitive impairment (MCI) or is at risk of developing AD, the method comprises the following steps:
a) optionally, providing a sample to be tested from a subject,
b) contacting the sample from the subject with a reagent for detecting the presence and/or level of Apo*3F in the sample from the subject,
c) detecting the presence and/or level of the Apo*3F in the sample from the subject,
wherein the presence and/or level of the Apo*3F in the sample from the subject indicates that the subject suffers from AD or AD-derived MCI, wherein the Apo*3F is an Aβ oligomer specifically bound by the antibody 3F, and has a molecular weight of approximately 588 kDa based on size exclusion chromatography (SEC) analysis, and the light and heavy chain CDR sequences of the antibody 3F are as set forth in SEQ ID NOs: 17-22, respectively.

In some embodiments, the subject is a patient suspected of having AD or AD-derived MCI, preferably, the subject is a human, a non-human primate, a cat or a dog.

In some embodiments, the diagnostic method further comprises a step of clinical neuropsychological and neuroimaging assessment, preferably, the neuroimaging assessment is Aβ-PET scanning imaging and/or tau-PET. More preferably, the neuroimaging assessment is 3F-PET scanning imaging, i.e., the antibody 3F-based immunoPET imaging, for identifying and detecting the presence and/or level of the Apo*3F in the brain of a subject.

In some embodiments, the diagnostic method further comprises detecting changes in the tau levels in the sample from the subject, preferably, the tau level is the total tau level or the phosphorylated tau level.

Another aspect of the present invention relates to a kit for diagnosing whether a subject suffers from early and mid-late stage AD or AD-derived MCI or is at risk of suffering from AD, comprising a reagent for detecting the presence and/or level of Apo*3F in a sample of the subject, wherein the Apo*3F is an Aβ oligomer specifically bound by the antibody 3F, and has a molecular weight of approximately 588 kDa based on size exclusion chromatography analysis, and the light and heavy chain CDR sequences of the antibody 3F are as set forth in SEQ ID NOs: 17-22, respectively.

In some embodiments, the sample from the subject is a sample obtained from the subject on-site when performing the diagnosis. In some embodiments, the sample from the subject is a sample obtained from the subject in previous diagnostic procedure.

In some embodiments, the size exclusion chromatography analysis is performed using a Superdex 200 10/300 GL molecular exclusion chromatography column.

In some embodiments, the Apo*3F is present in a cerebrospinal fluid sample obtained from the subject. In some embodiments, the cerebrospinal fluid sample of the subject is analyzed using immunoprecipitation and size exclusion chromatography and the molecular weight of the Apo*3F is determined.

In some embodiments, the method or kit involves or comprises an enrichment reagent for the Aβo*3F, wherein the enrichment reagent for the Apo*3F includes a binding agent specifically binding to the Aβo*3F, preferably, the binding agent specifically binding to the Apo*3F is an antibody specifically binding to the Aβo*3F, preferably, the antibody is a monoclonal antibody, a polyclonal antibody or an antigen-binding fragment thereof, more preferably, the antigen-binding fragment is selected from the group consisting of scFv, F(ab')2, Fab2, Fab, Fab', Fv, Fd, dAb, camelid antibody, nanobody, diabody and bispecific antibody. The Apo*3F in the sample from the subject can be effectively enriched using the enrichment reagent for the Apo*3F for subsequent procedures.

In some embodiments, the reagent for detecting the presence and/or level of the Apo*3F comprises a first binding agent specifically binding to the Apo*3F or Aβ aggregates, preferably, the first binding agent specifically binding to the Apo*3F or Aβ aggregates is an antibody specifically binding to the Apo*3F or Aβ aggregates, preferably, the antibody is a monoclonal antibody, a polyclonal antibody or an antigen-binding fragment thereof, more preferably, the antigen-binding fragment is selected from the group consisting of scFv, F(ab')2, Fab2, Fab, Fab ', Fv, Fd, dAb, camelid antibody, nanobody, diabody and bispecific antibody. In some embodiments, the first binding agent specifically binding to the Apo*3F or Aβ aggregates is a scFv or a full-length antibody specifically binding to the Apo*3F or Aβ aggregates.

In some embodiments, the first binding agent is linked to a detection agent that allows for its detection.

In some embodiments, the Apo*3F can be detected and defined using the K98R mutant of the antibody 3F. The Aβ oligomers detected using the K98R mutant are consistent with the Aβ oligomers detected by the antibody 3F, both of which are the Apo*3F. However, the K98R mutant has a higher affinity for binding to the Apo*3F than the antibody 3F, and can more sensitively detect the presence or absence of the Apo*3F and can more sensitively diagnose neurodegenerative diseases such as AD.

In some embodiments, the first binding agent specifically binding to the Apo*3F or Aβ aggregates is the antibody 3F or K98R mutant thereof.

In some embodiments, the reagent for detecting the presence and/or level of the Apo*3F further comprises a second binding agent specifically binding to the first binding agent, preferably, the second binding agent is an antibody specifically binding to the first binding agent, preferably, the antibody specifically binding to the first binding agent is a monoclonal antibody, a polyclonal antibody or an antigen-binding fragment thereof, more preferably, the antigen-binding fragment is selected from the group consisting of scFv, F(ab')2, Fab2, Fab, Fab', Fv, Fd, dAb, camelid antibody, nanobody, double antibody and bispecific antibody, preferably, the antibody specifically binding to the first binding agent is linked to a detection agent that allows for its detection. In some embodiments, the antibody specifically binding to the first binding agent is a full-length antibody.

In some embodiments, the detection agent is selected from the group consisting of chemiluminescent label, electrochemiluminescent label, chromophore, fluorescent label, fluorescein-type label, umbelliferone, lissamine, cyanine, Texas Red, BODIPY FL-SE^{®} (Invitrogen) or an analog thereof, paramagnetic label, radioactive label, biotin, streptavidin/biotin, avidin/biotin, hapten, digoxin, metal complex, metal, enzyme, colloidal gold, and a combination thereof. In some embodiments, the detection agent is selected from the group consisting of chemiluminescent label, electrochemiluminescent label, chromophore, fluorescent label, radioactive label, enzyme, and a combination thereof. In some embodiments, the detection agent is chemiluminescent label or electrochemiluminescent label.

In some embodiments, the detection is selected from the group consisting of chemiluminescence assay, electrochemiluminescence assay, enzyme-linked immunosorbent assay, immunofluorescence assay, immunohistochemistry assay, immunochromatography assay, radioimmunoassay, single molecule immunoassay technology (Simoa), flow cytometry, cell sorting, immunoprecipitation assay, immunodiffusion assay, dot blot assay, Western blot, protein chip, positron emission tomography and/or single photon emission computed tomography, preferably, the kit includes reagents, materials, containers and/ or devices required for the detection selected from the group consisting of chemiluminescence assay, electrochemiluminescence assay, enzyme-linked immunosorbent assay, immunofluorescence assay, immunohistochemistry assay, immunochromatography assay, radioimmunoassay, single molecule immunoassay technology, flow cytometry, cell sorting, immunoprecipitation assay, immunodiffusion assay, dot blot assay, Western blot and/or protein chip, preferably, the enzyme-linked immunosorbent assay is selected from the group consisting of direct enzyme-linked immunosorbent assay, indirect enzyme-linked immunosorbent assay, direct sandwich enzyme-linked immunosorbent assay and indirect sandwich enzyme-linked immunosorbent analysis.

In some embodiments, the sample from the subject is selected from the group consisting of cells, tissues, organs and/or body fluids of the subject, preferably, the body fluid is selected from the group consisting of whole blood, plasma, serum, cerebrospinal fluid, lymph, saliva, synovial fluid, bronchoalveolar lavage fluid, sputum, ascites, urine, amniotic fluid, peritoneal fluid, pericardial fluid, semen and/or vaginal secretions, preferably, the body fluid is selected from the group consisting of whole blood, plasma, serum and/or cerebrospinal fluid.

In some embodiments, the enrichment agent or the first binding agent specifically binding to the Apo*3F is attached to a solid support.

In some embodiments, the method further comprises a step of detecting a control sample, and/or the kit further comprises a control sample, wherein the control sample is from a healthy subject or a subject not suffering from AD and AD-derived MCI.

In some embodiments, the antibody specifically binding to the Apo*3F is a polyclonal antibody and/or a monoclonal antibody or an antigen-binding fragment thereof obtained by immunization against the Aβo*3F, preferably, the antibody is a human antibody, a humanized antibody, a chimeric antibody, a mouse antibody, a rat antibody, a rabbit antibody, a goat antibody, a horse antibody, a sheep antibody or a non-human primate antibody.

In some embodiments, based on the MSD electrochemiluminescence assay, the concentration of the Apo*3F in the cerebrospinal fluid of healthy population is Aβ42o*3F: 80.44 ± 20.88 pg/ml, Aβ40o*3F: 24.35 ± 5.08 pg/ml; and/or the concentration of the Apo*3F in plasma is Aβ42o*3F: 71.63 ± 36.8 pg/ml, Aβ40o*3F: 2.24 ± 0.92 pg/ml; the concentration of the Apo*3F in plasma of MCI population is Aβ42o*3F: 112.93 ± 25.02 pg/ml, Aβ40o*3F: 4.64 ± 1.43 pg/ml; the concentration of the Apo*3F in the cerebrospinal fluid of AD patient population is Aβ42o*3F: 264.8 ± 42.26 pg/ml, Aβ40o*3F: 85.74 ± 10.62 pg/ml, and/or the concentration of the Apo*3F in plasma is Aβ42o*3F: 159.44 ± 36.8 pg/ml, Aβ40o*3F: 14.0 ± 5.59 pg/ml; or
based on the chemiluminescence assay, the concentration of Aβ42o*3F in the plasma of healthy population is 68.02 ± 39.17 pg/ml; the concentration of Aβ42o*3F in the plasma of population with MCI is 124.5 ± 12.57 pg/ml; the concentration of Aβ42o*3F in the plasma of AD patient population is 205.75 ± 50.96 pg/ml. In some embodiments, the MSD electrochemiluminescence assay is performed as described in Example 7 of the specification. In some embodiments, the chemiluminescence method is performed as described in Example 8 of the specification.

In some embodiments, the detection sensitivity is as low as 0.5 pg/ml based on the MSD electrochemiluminescence assay and chemiluminescence assay.

In some embodiments, the antibody specifically binding to the Apo*3F is a plurality of antibodies specific for different epitopes on the Aβo*3F, for example, 2, 3, 4, 5 or more antibodies that are specific for 2, 3, 4, 5 or more different epitopes on the Aβo*3F, respectively.

In some embodiments, the step of enriching the Apo*3F from the sample is performed using immunoprecipitation with an antibody specifically binding to the Apo*3F.

In other words, in the preliminary research, the present inventors used phage display technology to firstly screen out the fully human single-chain antibody W20 (see CN101463082A), and the antibody 3F (having the amino acid sequence as set forth in SEQ ID No. 23) is an improved form of the antibody 20. Compared with the antibody 20, the antibody 3F's affinity to Aβ oligomers is significantly improved, and the antibody 3F can more significantly inhibit the aggregation of Aβ and the neuronal cytotoxicity induced by Aβ oligomers, more effectively improve the cognitive and memory functions of AD model mice, and reduce pathological changes in the mouse brain. More significantly, the Aβ oligomers specifically recognized by this antibody are super toxic oligomers, they are the main toxic component in the Aβ oligomer mixture, have a strong pathogenic effect, and play an important role in the occurrence and development of AD. The highly toxic oligomer recognized by the antibody 3F is present in the CSF, blood and/or brain tissue of AD patients and AD-derived MCI patients, and its level shows extremely significant differences in the CSF, blood and/or brain of the three human groups, i.e., AD patients, MCI patients and healthy elderly patients, so that AD patients, MCI patients and healthy elderly people can be accurately distinguished. This toxic oligomer also exists in AD transgenic mice and is directly related to the pathogenesis of AD transgenic mice. In the present invention, the highly toxic Aβ oligomer recognized by the antibody 3F is called AβO*3F. It can be separated from the Aβ oligomer mixture by immunoprecipitation (with the antibody 3F). Its typical characteristics are Aβ high molecular weight oligomers, based on size exclusion chromatography (SEC) analysis, its molecular weight is approximately 588 kDa and its diameter is approximately 10 nm. It has a strong toxic effect on neurons, and its toxicity is more than 200 times more toxic than the Aβ oligomer mixture. The Apo*3F can activate microglia and/or astrocytes and secrete a large number of inflammatory factors.

### BRIEF DESCRIPTION OF THE DRAWINGS

Figure 1 shows a schematic diagram of immunoprecipitation preparation of the Aβo*3F, Aβ*6E10 and Aβ-ID.
Figure 2 shows the molecular weight and morphology of the Aβo*3F:
   A: the Aβ aggregation state detected by ThT;
   B: the binding of oligomers obtained under different incubation conditions with the antibodies 3F, A11 and 6E10 detected by Dot blot;
   C: the affinity of Aβos obtained from 0-4 days of 10 µM Aβ incubation with the antibody 3F;
   D: the sAβo*3F band distribution detected by Western blot;
   E: Size exclusion chromatography (SEC) analysis of the molecular weight of sAβo*3F; SEC analysis of sApo*3F and SEC standards (Superdex 200 10/300), SEC standards: 1. Thyroglobulin (669 kDa), 2. Ferritin (440 kDa), 3. Aldolase (158 kDa), 4. Conalbumin (75 kDa), 5. Ovalbumin (44 kDa), 6. Carbonic anhydrase (29 kDa);
   F: Fitting linear equation (Y) and correlation coefficient (r2) according to the molecular weight and elution volume of SEC standards;
   G: Western blot detection of mAβo*3F band distribution;
   H: SEC analysis of mAβo*3F molecular weight;
   I: Western blot detection of hAβo*3F band distribution.

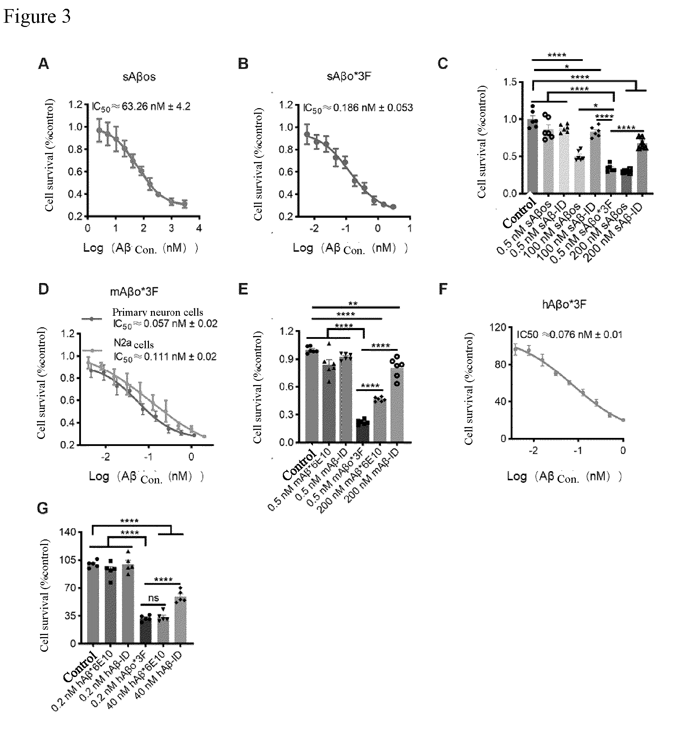

Figure 3 shows the neurotoxic effect of the Aβo*3F:
   A: IC50 of sAβos cytotoxicity to N2a cells;
   B: IC50 of sAβo*3F cytotoxicity to N2a cells;
   C: MTT method was used to compare the neurotoxicity of sAβos, sApo*3F and sAβ-ID to N2a cells;
   D: IC50 of mAβo*3F toxicity to N2a cells and primary neurons;
   E: MTT method was used to compare the cytotoxicity of mAβ*6E10, mAβo*3F and mAβ-ID on primary neurons;
   F: IC50 of hAβo*3F toxicity to primary neurons;
   G: MTT method was used to compare the cytotoxicity of hAβ*6E10, hAβo*3F and hAβ-ID to primary neurons.

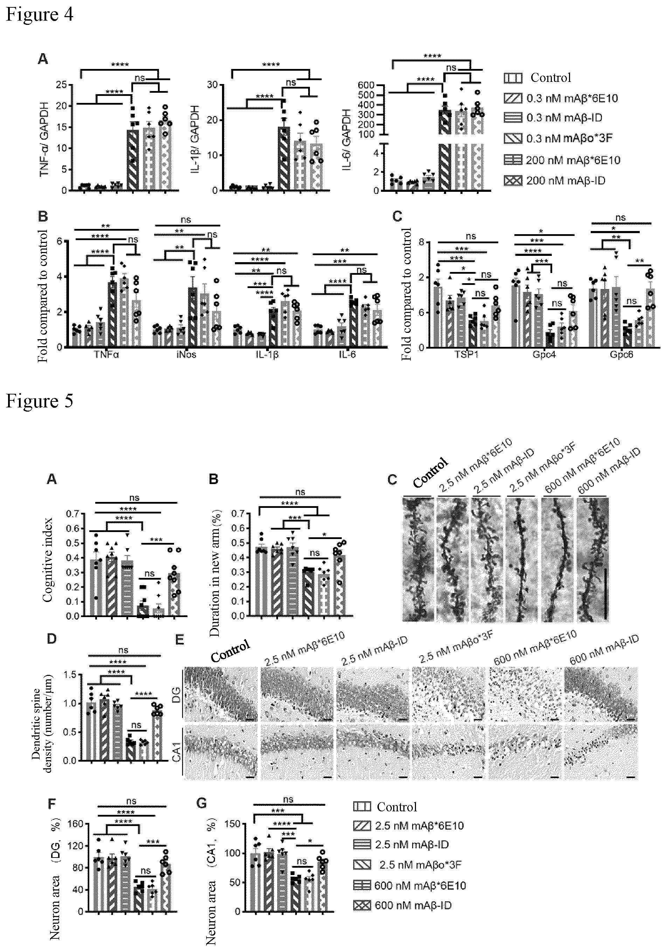

Figure 4 shows the effect of Apo*3F on the cytokine expression level of glial cells:
   A: Effects of mAβo*3F on TNF-α, IL-1β, and IL-6 expression levels in microglia;
   B: Effects of mAβo*3F on TNF-α, iNos, IL-1β, and IL-6 expression levels in primary astrocytes;
   C: Effect of mAβo*3F on TSP1, Gpc4 and Gpc6 expression levels in primary astrocytes.
Figure 5 shows the effects of the Apo*3F on mouse cognition function and its damaging effects on brain neurons:
   A: Cognitive index of mice in each group in the novel object recognition test;
   B: The duration of each group of mice in the new arm in the Y-maze test;
   C: Golgi staining to detect the density of dendritic spines in mouse neurons;
   D: Statistical analysis of dendritic spine density in C;
   E: Nissl staining to detect the number of neurons in mouse hippocampus, scale bar: 20 µm;
   F, G: The number of neurons in the DG area (F) and CA1 area (G) was statistically analyzed using Image J software.

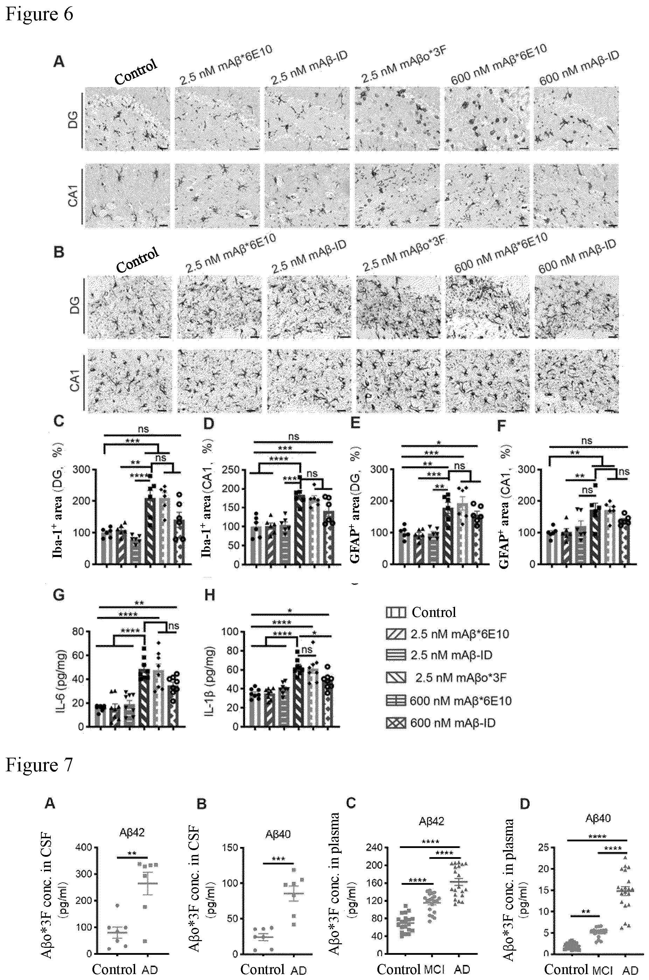

Figure 6 shows that the Apo*3F activates glial cells in the mouse brain to produce neuroinflammation:
   A: Immunohistochemistry assay to detect the activation of microglia in the DG and CA1 regions of the mouse hippocampus, scale bar: 20 µm;
   B: Immunohistochemistry assay to detect the activation of astrocytes in the DG and CA1 regions of the mouse hippocampus, scale bar: 20 µm;
   C, D: The area of Iba-1-positive microglia in the DG region (C) and CA1 region (D) was quantified using Image J software;
   E, F: The area of GFAP-positive astrocytes in the DG region (E) and CA1 region (F) was quantified using Image J software;
   G, H: The expression levels of IL-6 (G) and IL-1β (H) in the mouse hippocampus were detected by ELISA.
Figure 7 shows the electrochemiluminescence method for detecting the Apo*3F levels in CSF and plasma of AD patients:
   A, B: the corresponding levels of Aβ42 (A) and Aβ40 (B) in the Apo*3F isolated from CSF of AD patients and healthy population (Con) were detected by MSD method;
   C, D: the corresponding levels of Aβ42 (C) and Aβ40 (D) in the Apo*3F isolated from the plasma of patients with mild cognitive impairment (MCI), AD and healthy population (Con) were detected by MSD method.

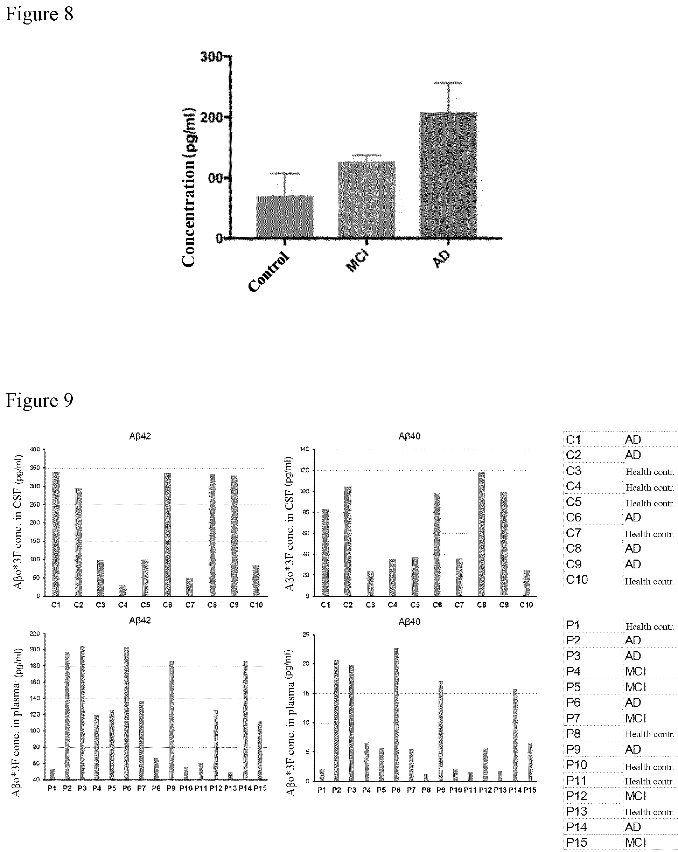

Figure 8 shows the Apo*3F levels in plasma of AD patients, MCI patients and healthy population detected with chemiluminescence assay.
Figure 9 shows AD diagnosis by detecting the Apo*3F levels in CSF or plasma samples.

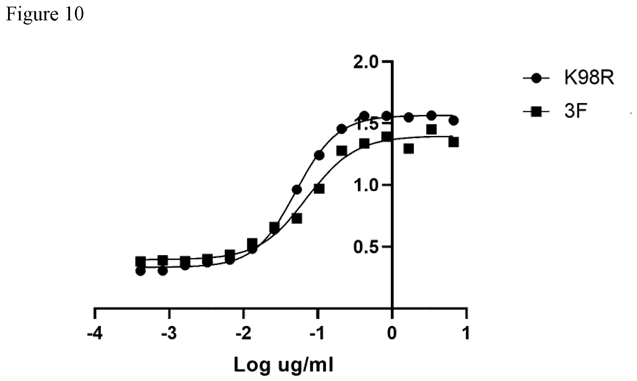

Figure 10 shows that after the K at position 98 of the antibody 3F heavy chain mutated to R, the affinity for binding to the Apo*3F was further improved, suggesting that the optimized single-chain antibody can be used to better diagnose the presence of the Apo*3F oligomers and further to better diagnose AD.

### DETAILED DESCRIPTION

### Definitions

Unless otherwise indicated, the terms used in the claims and specification are defined as shown below.

Unless otherwise defined herein, the scientific and technical terms used in conjunction with the methods and compositions of the present invention as described herein shall have the meanings commonly understood by those of ordinary skill in the art. In addition, unless the context otherwise requires, singular terms shall include plural terms, and plural terms shall include singular terms. Typically, the nomenclature and techniques used in conjunction with the following fields are those well known and commonly used in the art: biochemistry, immunology enzymology, molecular and cell biology, microbiology, genetics, and polypeptide chemistry, as described herein.

The methods and techniques described herein are generally performed according to conventional methods well known in the art and as described in various general and more specific references that are cited and discussed throughout the present specification, unless otherwise indicated. See, e.g., Sambrook et al., Molecular Cloning: A Laboratory Manual, 2nd ed., Cold Spring Harbor Laboratory Press, Cold Spring Harbor, NY (1989); Ausubel et al., Current Protocols in Molecular Biology, Greene Publishing Associates (1992, and supplements through 2002); Harlow and Lane, Antibodies: A Laboratory Manual, Cold Spring Harbor Laboratory Press, Cold Spring Harbor, NY (1990); Taylor and Drickamer, Introduction to Glycobiology, Oxford Univ. Press (2003); Worthington Enzyme Manual, Worthington Biochemical Corp., Freehold, NJ; Handbook of Biochemistry: Section A Proteins, Volume I, CRC Press (1976); Handbook of Biochemistry: Section A Proteins, Volume II, CRC Press (1976); Essentials of Glycobiology, Cold Spring Harbor Laboratory Press (1999).

All the publications, patents, and other references mentioned herein are expressly incorporated by reference in their entirety.

Unless otherwise indicated, the following terms shall be understood to have the following meanings.

The term "at risk of developing Alzheimer's disease" as used herein refers to having a risk of developing Alzheimer's disease, for example due to family history, aging, unhealthy lifestyle such as drinking, and the like.

The term "Aβ-PET scanning imaging" as used herein refers to positron emission tomography on β-amyloid.

The term "tau PET scanning imaging" as used herein refers to positron emission tomography on tau protein.

The term "3F-PET scanning imaging" as used herein refers to immunopositron emission tomography based on the antibody 3F. The term "Aβo*3F" as used herein refers to an Aβ oligomer specifically bind to the antibody 3F, and based on size exclusion chromatography (SEC) analysis, its molecular weight is about 588 kDa, and the light and heavy chain CDR sequences of the antibody 3F are as set forth in SEQ ID NOs: 17-22, respectively..

The term "Aβ42o*3F" as used herein refers to the Aβ42 oligomer portion in the Apo*3F.

The term "Aβ40o*3F" as used herein refers to the Aβ40 oligomer portion in the Apo*3F.

In some embodiments, the chemiluminescent label of the present disclosure is selected from the group consisting of alkaline phosphatase, horseradish peroxidase, isoluminol and its derivatives, acridinium ester and its derivatives.

In some embodiments, the chromophore of the present disclosure is selected from tris-bipyridine ruthenium.

In some embodiments, the fluorescent label of the present disclosure is selected from the group consisting of rare earth chelates and derivatives thereof, isothiocyanates, rhodamine, phycobilin, phycoerythrin, phycocyanin, allophycocyanin, fluorescein isothiocyanate, and AlexaFluor dyes.

In some embodiments, the fluorescein-type label of the present disclosure is selected from the group consisting of luciferin, luciferase, such as firefly luciferase, and bacterial luciferase.

In some embodiments, the paramagnetic label includes, but is not limited to, a compound comprising one selected from the group consisting of aluminum (Al), barium (Ba), calcium (Ca), cerium (Ce), dysprosium (Dy), erbium (Er), europium (Eu), gadolinium (Gd), holmium (Ho), iridium (Ir), lithium (Li), magnesium (Mg), manganese (Mn), molybdenum (Mo), neodymium (Nd), osmium (Os), oxygen (O), palladium (Pd), platinum (Pt), rhodium (Rh), ruthenium (Ru), samarium (Sm), sodium (Na), strontium (Sr), terbium (Tb), thulium (Tm), tin (Sn), titanium (Ti), tungsten (W), vanadium (V) and zirconium (Zi) and in particular Co⁺², CR⁺², Cr⁺³, Cu⁺², Fe⁺², Fe⁺³, Ga⁺³, Mn⁺³, Ni⁺², Ti⁺³, V⁺³ and V⁺⁴ paramagnetic ions.

In some embodiments, the radiolabel is selected from the group consisting of technetium-99m, iodine-123, iodine-125, iodine-131, indium-111, fluorine-19, carbon-13, nitrogen-15, oxygen-17, phosphorus-32, sulfur-35, deuterium, tritium, rhenium-186, rhenium-188, and yttrium-90.

In some embodiments, the hapten is selected from polysaccharide or lipoid.

In some embodiments, the metal complex is selected from tris-bipyridine ruthenium.

In some embodiments, the metal is selected from ruthenium.

In some embodiments, the enzyme of the present disclosure is selected from the group consisting of horseradish peroxidase, alkaline phosphatase, β-galactosidase, acetylcholinesterase, β-glucuronidase, β-D-glucosidase, urease, hexokinase, malic enzyme, glucose-6-phosphate dehydrogenase, sucrase, glucoamylase, lysozyme, sugar oxidase, heterocyclic oxidase, streptavidin-β-D-galactopyranose conjugate and/or streptavidin-horseradish peroxidase conjugate.

In some embodiments, tris-bipyridine ruthenium is used for electrochemiluminescent assay.

In some embodiments, the detection agent for the biotinylated detection antibody is avidin, streptavidin-HRP, or streptavidin-β-D-pyranose galactose (SBG). In certain embodiments, the readout of the detection agent is fluorometric or colorimetric, for example, but not limited to, using tetramethylbenzidine and hydrogen peroxide for the readout. In certain embodiments, if the detection agent is streptavidin-HRP, the readout can be colorimetric by using tetramethylbenzidine and hydrogen peroxide. Alternatively, in certain embodiments, resorufin β-D-pyranose galactoside can be used as the readout. For example, but not limited to, if the detection agent is SBG, the readout can be fluorometric by using resorufin β-D-pyranose galactoside.

In certain embodiments, the detection agent, such as SBG, can be used at a concentration of about 50 to about 500 pM. For example, but not limited to, the detection agent can be used at a concentration of about 50 to about 100 pM, about 50 to about 150 pM, about 50 to about 200 pM, about 50 to about 250 pM, about 50 to about 300 pM, about 50 to about 350 pM, about 50 to about 400 pM, about 50 to about 450 pM, about 100 to about 500 pM, about 150 to about 500 pM, about 200 to about 500 pM, about 250 to about 500 pM, about 300 to about 500 pM, about 350 to about 500 pM, about 400 to about 500 pM, about 450 to about 500 pM, about 100 to about 400 pM, or about 200 to about 400 pM. In certain embodiments, the detection agent can be used at a concentration of about 100 pM to about 400 pM, for example, SBG can be used at a concentration of about 110 pM, about 155 pM, or about 310 pM. In certain embodiments, SBG can be used at a concentration of about 310 pM. In certain embodiments, the detection agent, for example, HRP, can be used at a dilution of about 1/10 to about 1/1000. For example, but not limited to, the detection agent can be used at a dilution of about 1/10 to about 1/100, about 1/10 to about 1/500, about 1/100 to about 1/1000, or about 1/500 to about 1/1000. In certain embodiments, the detection agent can be used at a dilution of about 1/100 to about 1/1000, for example, HRP can be used at a dilution of about 1/100 or about 1/500. The concentration of the detection agent can be easily determined by those skilled in the art according to the type of the detection agent without exceeding routine experiments.

In some embodiments, as the enzyme color development matrix, for example, when horseradish peroxidase (HRP) is selected as the enzyme label, a solution containing 3-amino-9-ethylcarbazole, 5-aminosalicylic acid, 4-chloro-1-naphthol, o-phenylenediamine, 2,2'-azino-bis(3-ethylbenzothiazoline-6-sulfonic acid), 3,3-diaminobenzidine, 3,3',5,5'-tetramethylbenzidine, anisidine or 3,3'-dimethoxybenzidine can be used as the matrix. And, when alkaline phosphatase is selected as the enzyme label, a solution containing 5-bromo-4-chloro-3-indolyl phosphate, nitro blue tetrazolium or p-nitrophenol phosphate can be used as the matrix. And, when selecting β-D-glucosidase as the enzyme label, a solution containing o-nitrophenyl-β-D-galactoside or 5-bromo-4-chloro-3-indolyl-β-D-pyranogalactoside can be used as the matrix. In addition, various enzymes and enzyme color development substrates known in the art can be used.

In certain embodiments, the methods of the present disclosure may comprise blocking the first binding agent with a blocking buffer. In certain embodiments, the blocking buffer may include PBS, bovine serum albumin (BSA), and/or a biocide, such as ProClin^{™} (Sigma-Aldrich, Saint Louis, MO). In certain embodiments, the method may include multiple washing steps. In certain embodiments, the solution used for washing is typically a buffer (e.g., a "washing buffer"), such as, but not limited to, a PBS buffer including a detergent (e.g., Tween 20). For example, but not limited to, the first binding agent may be washed off after blocking and/or the sample may be separated from the first binding agent to remove unbound material (e.g., by washing).

In some embodiments, the diagnostic method of the present invention have a detection sensitivity as low as about 0.5 pg/ml, for example, about 1.0 pg/ml, 1.5 pg/ml, 2.0 pg/ml, 2.5 pg/ml, 3.0 pg/ml, 3.5 pg/ mL, 4.0 pg/ml, 4.5 pg/ml, 5.0 pg/ml, 5.5 pg/ml, 6.0 pg/ml, 7.5 pg/ml, 8.0 pg/ml, 8.5 pg/ml, 9.0 pg/ml, 9.5 pg/ml, 10.0 pg/ml, 15 pg/ml, 20 pg/ml, 25 pg/ml, 30 pg/ml, 50 pg/ml, 100 pg/ml, 150 pg/ml, 200 pg/ml, 250 pg/ml, 500 pg/ml, 750 pg/mL, 1000 pg/ml, 1500 pg/ml, 2000 pg/ml, 3000 pg/ml, 5000 pg/ml or higher.

In some embodiments, the kit of the present invention includes a first container containing an enrichment reagent or a first binding agent specifically binding to the Apo*3F.

In some embodiments, the kit of the present invention includes a second container comprising a second binding agent specifically binding to the first binding agent.

The term "the second binding agent specifically binding to the first binding agent" as used herein means that the second binding agent specifically binds to the first binding agent, such as an antibody of the antibody class. For example, if the first binding agent is mouse anti-Aβo*3F IgG, the second binding agent can be rabbit anti-mouse IgG.

In some embodiments, the kit of the present invention includes a third container containing a control sample.

In some embodiments, the kit of the present invention includes one or more other containers, which contain other reagents and materials required for performing the detection.

In some embodiments, the enrichment agent or the first binding agent specifically binding to the Aβo*3F or the second binding agent specifically binding to the first binding agent is attached to a solid support. In some embodiments, the solid support is selected from the group consisting of polystyrene, polyethylene, polypropylene, polyester, polyacrylonitrile, fluororesin, agarose, cellulose, nitrocellulose, plasma substitute, cross-linked glucose, Sepharose, liposomes, carboxymethyl cellulose, polyacrylamide, polystyrene, quartz, filter paper, ion exchange resin, plastic film, plastic tube, poly(methyl vinyl ether/maleic acid) copolymer, amino acid copolymer, ethylene-maleic acid copolymer, nylon, metal, glass, glass beads and magnetic particles. In addition, other solid supports include cell culture plates, enzyme-linked immunosorbent assay plates, electrochemiluminescence assay plates, tubes and polymeric membranes. The above-mentioned solid support can be any shape such as spherical (microbeads), cylindrical (inner surface of test tubes or wells), flat (sheets, test strips), etc.

In some embodiments, the kit of the present disclosure is a kit for performing enzyme-linked immunosorbent assay (ELISA), which includes some or all of the reagents, materials, containers and/or devices required for performing ELISA. Those skilled in the art would know how to prepare the corresponding ELISA kit based on the reagents for detecting the presence and/or level of the Apo*3F in a sample from a subject as disclosed herein. In some embodiments, the ELISA kit includes pre-coated Apo*3F antibodies, HRP-labeled Aβ antibodies, standards, washing solutions, substrates, and the like.

In some embodiments, the kit of the present disclosure is a kit for performing immunofluorescence assay, which includes some or all of the reagents, materials, containers and/or devices required for performing immunofluorescence assay. Those skilled in the art would know how to prepare a corresponding immunofluorescence assay kit based on the reagents for detecting the presence and/or level of the Apo*3F in a sample from a subject as disclosed herein. In some embodiments, the immunofluorescence assay kit is electrochemiluminescent immunoassay. In some embodiments, the immunofluorescence assay kit is time-resolved immunofluorescence assay. In some embodiments, the immunofluorescence assay kit includes an electrochemiluminescent plate pre-coated with an antibody specific for Aβo*3F, ruthenium-labeled Aβ antibodies, standards, reading buffer, and the like.

In some embodiments, the kit of the present disclosure is a kit for performing colloidal gold assay, which includes some or all of the reagents, materials, containers and/or devices required for performing colloidal gold assay. Those skilled in the art would know how to prepare a corresponding colloidal gold assay kit based on the reagents for detecting the presence and/or level of the Apo*3F in a sample from a subject as disclosed herein. In some embodiments, the colloidal gold assay kit is a colloidal gold test strip. In some embodiments, the colloidal gold assay kit includes colloidal gold-labeled Apo*3F antibodies, Aβ antibodies, control antibodies, and the like.

In some embodiments, the kit of the present disclosure is a kit for performing radioimmunoassay, which includes some or all of the reagents, materials, containers and/or devices required for performing radioimmunoassay. Those skilled in the art would know how to prepare a corresponding radioimmunoassay kit based on the reagents for detecting the presence and/or level of the Apo*3F in a sample from a subject as disclosed herein. In some embodiments, the radioimmunoassay kit includes radioisotope-labeled Apo*3F antibodies, the Apo*3F standards, etc.

The term "antibody" as used herein refers to an immunoglobulin molecule or a fragment of an immunoglobulin molecule which has the ability to bind to an epitope of an antigen. Naturally occurring antibodies typically comprise a tetramer which is usually composed of at least two heavy (H) chains and at least two light (L) chains. Immunoglobulins include the following isotypes: IgG, IgA, IgM, IgD, and IgE, and the corresponding heavy chains thereof are µ chain, δ chain, γ chain, α chain, and ε chain, respectively. The same class of Igs can be divided into different subclasses according to the differences in the amino acid compositions of the hinge regions and the numbers and positions of the heavy chain disulfide bonds, such as IgG can be divided into IgG1, IgG2, IgG3, IgG4 subtypes, and IgA can be divided into IgA₁ and IgA₂ subtypes. Light chains can be divided into κ chains and λ chains according to the differences in constant regions. The antibody of the invention may be of any isotype. The choice of isotype typically will be guided by the desired effector functions, such as ADCC induction. Exemplary isotypes are IgG1, IgG2, IgG3, and IgG4. Either of the human light chain constant domains, κ or λ, may be used. If desired, the class of the antibody of the present invention may be switched by known methods. For example, an antibody of the present invention that was originally IgG may be class switched to an IgM antibody of the present invention. Further, class switching techniques may be used to convert one IgG subclass to another, for instance from IgGl to IgG2. Thus, the effector function of the antibodies of the present invention may be changed by isotype switching to, e.g., an IgG1, IgG2, IgG3, IgG4, IgD, IgA, IgE, or IgM antibody for various therapeutic uses, provided that the C1q binding activity of the antibody is reduced or eliminated. In some embodiments, the antibody of the present invention is an IgM or an antibody of IgG1, 2, 3 or 4 type. An antibody is said to be of a particular isotype if its amino acid sequence is most homologous to that isotype, relative to other isotypes.

Herein, the term "antibody" is used in the broadest sense thereof, and refers to a protein comprising an antigen-binding site, encompassing natural and artificial antibodies of various structures, including but not limited to intact antibodies and antigen-binding fragments of antibodies.

A "variable region" or "variable domain" is a domain of a heavy or light chain of an antibody involving in the binding of the antibody to its antigen. Each heavy chain of an antibody consists of a heavy chain variable region (herein referred to as VH) and a heavy chain constant region (herein referred to as CH), wherein the heavy chain constant region usually consists of 3 domains (CH1, CH2 and CH3). Each light chain consists of a light chain variable region (herein referred to as VL) and a light chain constant region (herein referred to as CL). The variable regions of the heavy and light chains are typically responsible for antigen recognition, while the constant domains of the heavy and light chains can mediate the binding of the immunoglobulin with host tissues or factors (including various cells of the immune system (e.g., effector cells), Fc receptors and the first component of the classical complement system (C1q)). The heavy and light chain variable regions comprise binding regions interacting with an antigen. The VH and VL regions can be further subdivided into hypervariable regions (HVRs) called "complementarity determining regions (CDRs)", which are interspersed with more conserved regions called "framework regions (FRs)". Each VH or VL consists of three CDR domains and four FR domains, arranged from the amino-terminus to the carboxy-terminus in the following order: FR1 - CDR1 - FR2 - CDR2 - FR3 - CDR3 - FR4.

The terms "complementarity determining region" or "CDR region" or "CDR" (used interchangeably herein with the hypervariable region "HVR") refer to a region in the antibody variable domains which is hypervariable in sequence and forms a loop structurally determined ("a hypervariable loop") and/or comprises antigen-contacting residues ("antigen-contacting points"). The CDRs are primarily responsible for binding to an epitope. Herein, the three CDRs of the heavy chain are referred to as HCDR1, HCDR2 and HCDR3, and the three CDRs of the light chain are referred to as LCDR1, LCDR2 and LCDR3.

It should be noted that the boundaries of the CDRs of the variable regions of the same antibody obtained based on different numbering systems may be different from each other. That is, the CDR sequences of the variable regions of the same antibody defined under different numbering systems are somewhat different from each other. Thus, when defining an antibody with the particular CDR sequences as defined in the invention, the scope of said antibody also covers antibodies of which the variable region sequences comprise said particular CDR sequences, but due to the different numbering system(s) used (such as different numbering system rules or combinations thereof), the CDR boundaries claimed by them might be different from the specific CDR boundaries defined in the invention.

The terms "mAbs", "monoclonal antibodies" or "monoclonal antibody composition" refer to a preparation of antibody molecules of single molecular composition, and refer to antibodies obtained from a substantially homogeneous population of antibodies, i.e., the population comprising individual antibodies are identical except for the naturally occurring mutations that may be present in minor amounts. A conventional monoclonal antibody composition displays a single binding specificity and affinity for a particular epitope. In certain embodiments, a monoclonal antibody can be composed of more than one Fab domain thereby increasing the specificity to more than one target. The term "monoclonal antibody" or "monoclonal antibody composition" is not limited by any particular method of production (e.g., recombinant, transgenic, hybridoma, etc.).

The present invention also includes "bispecific antibodies", wherein the antibodies of the present invention are part of a bivalent or multivalent bispecific framework targeting more than one epitope (for example, the second epitope may include an epitope of an active transport receptor, so that the bispecific antibody will exhibit improved transcytosis across biological barriers (such as the blood-brain barrier)). Therefore, in other embodiments, the monovalent Fab of the antibody of the present invention can be linked to another Fab or scFv targeting a different protein to produce a bispecific antibody. Bispecific antibodies can have dual functions, such as the therapeutic function conferred by the present invention and the transport function that can bind to receptor molecules to enhance transfer across biological barriers (such as the blood-brain barrier). The terms "double antibody", "bifunctional antibody", "bispecific antibody" or "BsAb" refers to an antibody which has two different antigen binding sites, so that which can simultaneously bind to two target antigens, alternatively which plays a role in mediating another special function simultaneously besides the targeting effect of the antibody. The special function effector molecules mediated can also be toxins, enzymes, cytokines, radionuclides, etc. The two arms of the bispecific antibody binding to an antigen can be from Fab, Fv, ScFv or dSFv, etc.

The term "antigen-binding fragment of antibody" refers to a fragment, a part, a region or a domain of an antibody capable of binding to an epitope (e.g., obtainable by cleavage, recombination, synthesis, etc.). An antigen-binding fragment may comprise 1, 2, 3, 4, 5 or all 6 CDR domains of such antibodies and, while capable of binding to the epitope, it may exhibit different specificities, affinities or selectivities. Preferably, an antigen-binding fragment comprises all 6 CDR domains of said antibody. An antigen-binding fragment of an antibody may be a part of a single polypeptide chain (e.g., scFv) or comprise a single polypeptide chain, or may be a part of two or more polypeptide chains (each having an amino- and carboxy-terminus) (e.g., a bispecific antibody, a Fab fragment, a F(ab')₂ fragment, etc.) or comprise two or more polypeptide chains.

Examples of an antigen-binding fragment of the invention include (a) a Fab' or Fab fragment, a monovalent fragment consisting of the VL, VH, CL and CH1 domains; (b) F(ab')₂ fragments, bivalent fragments comprising two Fab fragments linked by a disulfide bond at the hinge domain; (c) an Fd fragment consisting of the VH and CH1 domains; (d) a Fv fragment consisting of VL and VH domains of a single arm of an antibody; (e) a single chain Fv (scFv), a recombinant protein formed by linking VH and VL domains of an antibody with a peptide linker by genetic engineering; (f) a dAb fragment (Ward et al., Nature, 341, 544 -546 (1989)), which consists essentially of a VH domain and also called domain antibodies (Holt et al., Trends Biotechnol., 2i(11): 484-90); (g) camelid or nanobodies (Revets et al., Expert Opin Biol Ther., 5(1): 111-24) and (h) an isolated complementarity determining region (CDR).

In some embodiments, the antibodies and antigen-binding fragments thereof of the present invention are single-chain antibodies. In some embodiments, the present invention provides single-chain Fv (scFv), wherein the heavy chain and light chain in the Fv of the antibody of the present invention are connected into a single peptide chain by a flexible peptide linker (typically about 10, 12, 15 or more amino acid residues). Methods for producing such antibodies are described in, for example, US 4,946,778; Pluckthun, The Pharmacology of Monoclonal Antibodies, Vol. 113, Rosenburg and Moore ed., Springer-Verlag, New York, pp. 269-315 (1994); Bird et al., Science, 242, 423-426 (1988); Huston et al., PNAS USA 85, 5879-5883 (1988) and McCafferty et al., Nature, 348, 552-554 (1990). Single-chain antibodies are monovalent if only a single VH and VL are used, bivalent if two VHs and VLs are used, or multivalent if more than two VHs and VLs are used.

In some embodiments, the antibodies and antigen-binding fragments thereof of the present invention are chimeric antibodies. The term "chimeric antibody" means that a portion of the heavy and/or light chains is identical or homologous to the corresponding sequences of an antibody from a particular species or belonging to a particular antibody class or subclass, while the rest of the chains is identical or homologous to the corresponding sequences of an antibody from another species or belonging to another antibody class or subclass, providing that they exhibit the desired biological activity. The invention provides the variable region antigen binding sequences from human antibodies. Thus, chimeric antibodies primarily focused herein include antibodies which comprise one or more human antigen-binding sequences (e.g., CDRs) and comprise one or more sequences from non-human antibodies, such as FR or C region sequences. Furthermore, chimeric antibodies described herein are antibodies comprising human variable region antigen-binding sequence from one antibody class or subclass and other sequences from another antibody class or subclass, such as FR or C region sequences.

In some embodiments, the antibodies and antigen-binding fragments thereof of the present invention are humanized antibodies. The term "humanized antibody" refers to an antibody in which CDR sequences from the germline of another mammalian species, such as a mouse, have been grafted among the human framework region sequences. Additional framework region modifications can be made within the human framework region sequences.

In some embodiments, the antibodies and antigen-binding fragments thereof of the invention are human antibodies or fully human antibodies. The terms "human antibody" or "fully human antibody" ("humAb" or "HuMab") refers to an antibody comprising variable and constant domains derived from human germline immunoglobulin sequences. The human antibodies of the invention may include amino acid residues not encoded by human germline immunoglobulin sequences (e.g., mutations introduced by random or site-specific mutagenesis *in vitro* or during gene rearrangement or by somatic mutation *in vivo*)*.*

Variant antibodies are also included within the scope of the invention. Accordingly, variants of the sequences recited in the invention are also included within the scope of the invention. Other variants of antibody sequences with improved affinity can be obtained by using methods known in the art and these variants are also included within the scope of the invention. For example, amino acid substitutions can be used to obtain antibodies with further improved affinity. Alternatively, codon optimization of nucleotide sequences can be used to improve the translation efficiency of expression systems in antibody production.

Such variant antibody sequences have 70% or more (e.g., 80%, 85%, 90%, 95%, 97%, 98%, 99% or more) sequence homology to the sequences recited in the invention. Such a sequence homology is obtained by calculation relative to the full length of the reference sequence (i.e., the sequence recited in the invention).

The numbering of amino acid residues in the invention is according to IMGT^{®}, the international ImMunoGeneTics information system^{®} or Kabat, E. A., Wu, T. T., Perry, H. M., Gottesmann, K. S. & Foeller, C. (1991). Sequences of Proteins of Immunological Interest, 5th edit., NIH Publication no. 91-3242 U.S. Department of Health and Human Services; Chothia, C. & Lesk, A. M. (1987). Canonical Structures For The Hypervariable domains Of Immunoglobulins. J. Mol. Biol. 196, 901-917. Unless otherwise specified, the numbering of amino acid residues in the invention is according to the Kabat numbering system.

An antibody or an antigen-binding fragment thereof is said to "specifically" bind a region of another molecule (*i.e.,* an epitope) if it reacts or associates more frequently, more rapidly, with greater duration and/or with greater affinity or avidity with that epitope relative to alternative epitopes. In some embodiments, the antibody or antigen-binding fragment thereof of the invention binds to an amyloid protein, particularly its toxic form, with an affinity of at least 10⁻⁷ M, such as 10⁻⁸ M, 10⁻⁹ M, 10⁻¹⁰ M, 10⁻¹¹ M or higher. Preferably, the antibody or antigen-binding fragment thereof binds under physiological conditions (e.g., *in vivo*)*.* Therefore, specifically binding to an amyloid protein, particularly its toxic form, refers to the ability of the antibody or antigen-binding fragment thereof to bind to an amyloid protein, particularly its toxic form, with the above-mentioned specificity and/or under such conditions. Methods suitable for determining such a binding are known in the art.

The term "binding" in the context of the binding of an antibody to a predetermined antigen typically refers to binding with an affinity corresponding to a KD of about 10⁻⁶ M or less, which is at least ten-fold lower, such as at least 100-fold lower, for instance at least 1,000-fold lower than its affinity for binding to a non-specific antigen (e.g., BSA, casein) other than the predetermined antigen or a closely-related antigen.

As used herein, the term "kd" (sec -1 or 1/s) refers to the dissociation constant of a particular antibody-antigen interaction. Said value is also referred as the k_{off} value.

As used herein, the term "ka" (M-1 x sec-1 or 1/Msec) refers to the association rate constant of a particular antibody-antigen interaction.

As used herein, the term "KD" (M) refers to the dissociation equilibrium constant of a particular antibody-antigen interaction and is obtained by dividing the kd by the ka.

As used herein, the term "KA" (M-1 or 1/M) refers to the association equilibrium constant of a particular antibody-antigen interaction and is obtained by dividing the ka by the kd.

Antibodies of the invention are produced by any technique known in the art, such as, but not limited to, any chemical, biological, genetic, or enzymatic technique, alone or in combination. In general, if knowing the amino acid sequence of a desired sequence, one skilled in the art can readily generate such an antibody by standard techniques used to generate polypeptides. For example, these antibodies can be synthesized using well-known solid-phase methods, preferably using a commercially available peptide synthesis apparatus (such as that manufactured by Applied Biosystems, Foster City, California) and following the manufacturer's instructions. Alternatively, antibodies of the invention can be synthesized by recombinant DNA techniques well known in the art. For example, after incorporating a DNA sequence encoding an antibody into an expression vector and introducing these vectors into eukaryotic or prokaryotic host cells suitable for expressing the desired antibodies, antibodies as DNA expression products can be obtained, which can then be isolated from the host cells using known techniques.

The antibodies and antigen-binding fragments thereof of the present invention can be modified by including any "suitable" number of modified amino acids and/or in combination with coupling substituents. Suitability in this context is generally determined by the ability to retain the amyloid protein, particularly its toxic form, selectivity and/or the amyloid protein, particularly its toxic form, specificity at least substantially associated with the non-derivatized parent antibody. The inclusion of one or more modified amino acids may be advantageous in, for example, increasing polypeptide serum half-life, reducing polypeptide antigenicity, or increasing polypeptide storage stability. Amino acid(s) are modified, for example, co-translationally or post-translationally during recombinant production (e.g., N-linked glycosylation at N-X-S/T motifs during expression in mammalian cells) or modified by synthetic means. Non-limiting examples of a modified amino acid include a glycosylated amino acid, a sulfated amino acid, a prenylated (e.g., farnesylated, geranyl-geranylated) amino acid, an acetylated amino acid, an acylated amino acid, a PEGylated amino acid, a biotinylated amino acid, a carboxylated amino acid, a phosphorylated amino acid, and the like. References adequate to guide one of skill in the modification of amino acids are replete throughout the literature, see e.g., Walker (1998) Protein Protocols On CD-Rom, Humana Press, Totowa, NJ. The modified amino acid may, for instance, be selected from a glycosylated amino acid, a PEGylated amino acid, a farnesylated amino acid, an acetylated amino acid, a biotinylated amino acid, an amino acid conjugated to a lipid moiety, or an amino acid conjugated to an organic derivatizing agent.

The antibodies and antigen-binding fragments thereof of the present invention can also be chemically modified by covalent coupling to a polymer to increase its circulation half-life. Exemplary polymers and methods to attach them to peptides are illustrated in for instance US 4,766,106; US 4,179,337; US 4,495,285 and US 4,609,546. Additional illustrative polymers include polyoxyethylated polyols and polyethylene glycol (PEG) (e.g., a PEG with a molecular weight of between about 1,000 and about 40,000 D, such as between about 2,000 and about 20,000 D, e.g., about 3,000-12,000 D).

Antibodies and antigen-binding fragments thereof of the invention can be produced in different cell lines, such as human cell lines, non-human mammalian cell lines, and insect cell lines, such as CHO cell lines, HEK cell lines, BHK-21 cell lines, murine cell lines (such as myeloma cell line), fibrosarcoma cell line, PER.C6 cell line, HKB-11 cell line, CAP cell line, and HuH-7 human cell line (Dumont et al., 2015, Crit Rev Biotechnol., Sep. 18, 1-13., the contents of which are incorporated herein by reference).

The antibodies of the invention are suitably separated from the culture medium by conventional immunoglobulin purification methods, such as Protein A-Sepharose, hydroxyapatite chromatography, gel electrophoresis, dialysis, or affinity chromatography.

The term "subject" refers to warm-blooded animal, preferably, mammal (including human, livestock and farm animal, zoo animal, sports animal or pet animal, such as dog, cat, cattle, horse, sheep, pig, goat, rabbit, etc.), more preferably, human. In one embodiment, the subject can be a "patient", that is, warm-blooded animal, more preferably, human, who is waiting to receive or is receiving medical care or will be the subject of medical procedure or disease development monitoring. In one embodiment, the subject is an adult (e.g., subject over 18 years old). In another embodiment, the subject is a child (e.g., subject under 18 years old). In one embodiment, the subject is an elderly person who is older than 55, 60, 65, 70, 75, 80, 85, 90 years old or older. In one embodiment, the subject is a male. In another embodiment, the subject is a female.

The term " healthy subject" refers to a subject not suffering from any disease or condition or to a subject not suffering from any neurodegenerative disease.

The term "subjects not suffering from Alzheimer's disease" refers to subjects who are confirmed by regular medical procedures to be not suffering from Alzheimer's disease, such as subjects who are not suffering from mild cognitive impairment (MCI) and AD dementia as determined according to the criteria established by the United States National Institute on Aging and the Alzheimer's Association. In some embodiments, the subjects not suffering from Alzheimer's disease are healthy subjects.

The technical solutions of the present invention will be specifically described below by examples, which are descriptive and illustrative, and are not meant to be limiting. The reagents used in the following examples, if not specifically noted, can be easily purchased from reagent companies such as Sigma Aldrich and Merck, and the test methods, if not specifically noted, can be found in textbooks such as Sambrook, J., Fritsch, EF and Maniatis, T. (1989) Molecular Cloning: A Laboratory Manual. Cold Spring Harbor Press, New York.

### Examples

### Example 1 Obtaining of Aβ oligomers specifically bound by the antibody 3F

### 1.1 Experimental Materials and Methods

### 1.1.1 Experimental Materials

Protein A Magnetic Beads: Bio-RAD, #1614833
Protein G Magnetic Beads: Bio-RAD, #1614023
Protein A/G Agarose Gel: Abmart, #A10001S
The antibody 6E10: Biolegend, #803002
Human Aβ42 detection kit: Immuno-Biological Laboratories

### 1.1.2 Main solution

(1) 50 mM sodium hydroxide (pH 10.0);
(2) 20 mM phosphate buffer (PBS): pH 7.4;
(3) 0.1% PBST: PBS containing 0.1% Tween-20;
(4) 20 mM glycine elution buffer (pH 2.0);
(5) 1 M Tris neutralization solution: pH 10.0;

Note: The relevant reagents and solutions mentioned in Examples 2, 3, 4, 5 and 6 with no specified source are the same as those in Example 1.

### 1.2 Preparation of mouse brain homogenate

APP/PS1 mice were deeply anesthetized with sodium pentobarbital and sacrificed after transcardial perfusion with ice-cold PBS containing heparin (10 U/mL). 1 mL of RIPA strong lysis reagent (containing protease inhibitors and phosphatase inhibitors) was added to the brain tissue and lysed using Tissue Lyser II tissue homogenizer at a frequency of 30 Hz for 8 minutes, centrifuged at 4 °C, 14,000 rpm for 30 minutes, and collect the supernatant. The brain homogenate was added to 100 µ L of Protein A/G-agarose gel before immunoprecipitation, and incubated at 4 °C for 1 h to remove endogenous IgG in mouse brain homogenate. Then, the brain homogenate was added to Protein A magnetic beads cross-linked with APP antibody to remove endogenous APP in mouse brain.

### 1.3 Cross-linking antibodies with magnetic beads

First, the antibodies 3F and 6E10 were cross-linked with Protein A or Protein G magnetic beads, respectively. The specific cross-linking steps were as follows:
(1) Take 200 µL of Protein A or Protein G magnetic beads and wash them three times with 2 mL of 0.1% PBST. Mix the beads thoroughly each time and place on a magnetic rack. Discard the supernatant.
(2) Add 50 µg of the antibody 3F or 6E10 to the magnetic beads, shake at room temperature for 30 min to allow the beads to fully bind to the antibody, then wash three times with 0.1% PBST;
(3) Wash the magnetic beads with cross-linking buffer (0.2 M triethanolamine, pH 8.2) once, then dissolve 12 mg of DMP in 2 mL of cross-linking buffer, add to the magnetic beads, and shake at room temperature for 1 h. After the reaction is completed, discard the cross-linking solution, add 2 mL of blocking buffer (0.1 M ethanolamine, pH 8.2) to wash once, and then add 2 mL of blocking buffer again, and block at room temperature for 2 h;
(4) After blocking, wash the magnetic beads three times with PBS, then elute them once with 0.1 M glycine (pH 2.5), react for 5 min, and then wash the magnetic beads three times with 0.1% PBST. Immerse the magnetic beads in PBST containing 0.02% NaN3 and store them at 4 °C.

### 1.4 In vitro preparation of the Apo*3F

1 mg of Aβ42, Aβ40 or other forms of Aβ (Chinese Peptide Co., Ltd.) was dissolved in 1 mL of 100% hexafluoroisopropanol (HFIP), vortexed for 5 min, and ultrasonicated in a water bath for 10 min, then dispensed into EP tubes, the solvent was evaporated overnight, and stored at -20 °C. Before use, the HFIP-treated and aliquoted Aβ was dissolved in 50 mM NaOH at a concentration of 1 mg/mL, vortexed for 3-5 min, ultrasonicated for 1 min, and then diluted to 10 µM with pre-cooled PBS. Centrifuged at 21,000 g for 30-40 min at 4 °C, discarded the precipitate (about 5% of the starting volume), and Aβ monomers were obtained. The Aβ monomers were statically incubated at 25 °C for 2 days, and then incubated with Protein A magnetic beads cross-linked with the antibody 3F at 4 °C overnight. On the next day, the magnetic beads were washed three times with 0.1% PBST and eluted twice with 20-100 mM glycine (pH 2.0) for 3-5 min. The eluate was neutralized to pH7 with 1 M Tris to obtain sApo*3F (Aβo*3F prepared *in vitro*)*.*

1.5 Isolation and preparation of the Apo*3F from APP/PS1 mouse brain homogenate and AD patient CSF

To isolate and prepare Aβos specifically recognized by the antibody 3F (Aβo*3F), brain homogenates of APP/PS1 mice and CSF samples of AD patients (obtained from the First Affiliated Hospital of Zhengzhou University, with informed consent and approval from the Ethics Review Committee of the First Affiliated Hospital of Zhengzhou University) were incubated with Protein A magnetic beads cross-linked with the antibody 3F at 4°C overnight. The next day, the magnetic beads were washed three times with 0.1% PBST and then eluted twice with 20 mM-100 100 mM glycine (specifically 20 mM, pH2.0) for 3 min, and the eluate was neutralized to pH7 with 1 M Tris, and then the Apo*3F in the brains of APP/PS1 mice (mAβo*3F, the Apo*3F isolated from the brains of AD mice) or the Apo*3F extracted from the CSF of AD patients (hAβo*3F, the Apo*3F isolated from the cerebrospinal fluid of AD patients) was obtained. The Aβ aggregate mixture after the antibody 3F immunodepletion is called Aβ-ID, which are sAβ- ID (prepared *in vitro*), mAβ- ID (isolated and prepared from the brains of APP/PS1 mice) and hAβ- ID (isolated and prepared from the CSF of AD patients) and used as controls.

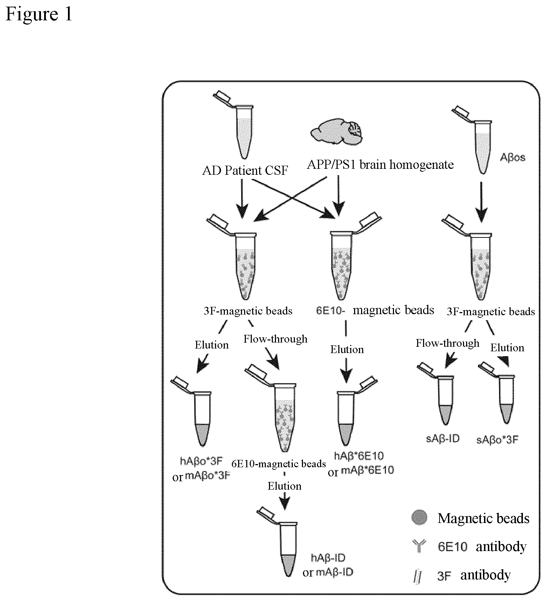

The Aβ aggregate mixture Aβ*6E10 was prepared by mixing APP/PS1 mouse brain homogenate or AD patient CSF with Protein G magnetic beads cross-linked with the 6E10 antibody, reacting at 4°C overnight, and then eluting twice with 20 mM-100 mM glycine (specifically 20 mM, pH 2.0) for 3-5 min (specifically 3 min), and the eluate was neutralized to pH7 with 1 M Tris, and then Aβo*6E10 in the brain of APP/PS1 mice (mAβo*6E10, Aβo*6E10 isolated from the brains of AD mice) or Aβo*6E10 extracted from the CSF of AD patients (hAβo*6E10, Aβo*6E10 isolated from the CSF of AD patients) was obtained (Figure 1). The concentration of Aβ obtained by immunoprecipitation was measured using an Aβ detection kit.

### Example 2 Characterization of molecular weight and morphology of the Apo*3F

### 2.1 Experimental Materials and Methods

### 2.1.1 Experimental Materials

Aβ polypeptide: Chinese Peptide Co., Ltd.;
Superdex 200 10/300 GL molecular exclusion chromatography: GE Healthcare;
The antibody A11: Invitrogen, #AHB0052;
Molecular exclusion chromatography protein marker: GE healthcare;
3-8% Tris-Acetate precast gel: Invitrogen;
Carbon film support copper mesh: Zhongke Keyi;
Uranyl acetate: Zhongke Keyi;
ECL chemiluminescence kit: Pierce Biotechnology;
ThT detection reagent: Sigma-Aldrich;
Nitrocellulose membrane: Millipore.

### 2.1.2 Experimental instruments

AKTA protein chromatograph: GE Healthcare Life Science, USA;
Protein electrophoresis instrument: Bio-rad, USA;
Protein transfer system: Bio-rad, USA;
HT7700 transmission electron microscope: Hitachi, Japan;
Safire2^{™} microplate reader: Tecan Group, Switzerland;
Amersham Imager 680 imaging system: GE, USA.

### 2.1.3 Main solutions

(1) 20 mM TBS buffer: Weigh 2.4 g Tris and 8 g NaCl, dissolve in deionized water, adjust its pH to 7.5 with dilute hydrochloric acid, and make up to 1 L.
(2) 0.1% TBST: Add 1 mL of Tween-20 to 1 L of TBS and stir for 20 min.
(3) ThT solution: Prepare 100X ThT stock solution (500 µM) with 50 mM PB (phosphate buffer, pH = 6.5) solution. Dilute to 1X with 50 mM PB (pH = 6.5) before use.
(4) 5% skim milk.
(5) Electrophoresis buffer: Dissolve 30 g Tris, 144 g Glycine, and 10 g SDS, make up to 1 L with deionized water, and dilute 10-fold before use.
(6) 5x non-reduced loading buffer (10 mL): 2 mL 10% SDS, 0.6 mL 1 M Tris-HCl (pH 6.8), 5 mL glycerol, 1 mL 1% bromophenol blue, and make up to 10 mL with deionized water.
(7) Transfer buffer: Dissolve 30 g Tris and 144 g Glycine in deionized water and make up to 1 L. Dilute 10-fold before use.

### 2.2 Preparation and characterization of Aβ monomers, oligomers, and fibers

The preparation of Aβ monomers, oligomers, and fibers was the same as in Item 1.4 above.

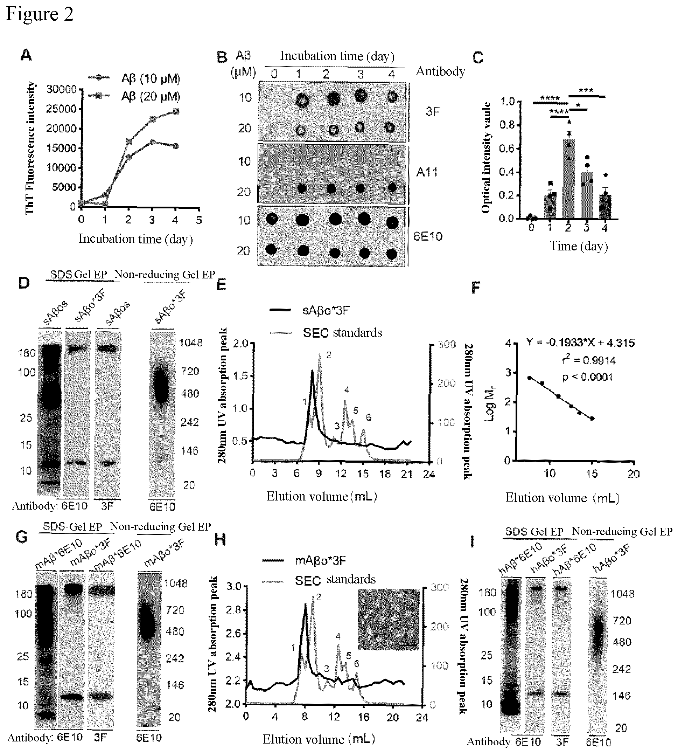

Aβ monomers were incubated at 25°C for 0-4 days, and the aggregation state of Aβ was detected by ThT every 24 h. The specific procedure was as follows: Aβ sample was diluted to 10 µM with PBS buffer, and then 190 µL of ThT detection solution and 10 µL of the sample to be tested were thoroughly mixed in a black plate, and the fluorescence was measured using a Safire2 ^{™} plate reader with an excitation wavelength of 440 nm and an emission wavelength of 480 nm. The results showed that as the incubation time increased, the ThT reading of the Aβ sample gradually increased, indicating that the Aβ monomers gradually aggregated into oligomers and fibers (Figure 2, panel A).

Dot blot experiments were used to evaluate the binding of different antibodies to Aβ monomers and aggregates. Aβ samples were spotted on nitrocellulose membranes and blocked with 5% skim milk for 1 h at room temperature. The membranes were incubated with different detecting antibodies for 1-2 h at room temperature, then washed three times with 0.1% TBST for 5 min each time, and HRP-labeled secondary antibodies were added for incubation at room temperature for 1 h. The membranes were washed three times with 0.1% TBST for 5 min each time, and ECL chemiluminescent solution was added to the membranes. The Amersham Imager 680 imaging system was used for color development, and the size and optical density of the blots were quantitatively analyzed using Image J software. The results showed that the antibody 6E10 is an antibody that binds widely to Aβ monomers, oligomers, and fibers. It can bind to 10 µM and 20 µM Aβ, and there is no significant difference in binding. Neither the antibody 3F nor A11 binds to Aβ monomers, and the antibodies 3F and A11 bind to different oligomer types. The antibody 3F mainly binds to 10 µM Aβos, while the antibody A11 mainly binds to 20 µM Aβos, indicating that the antibodies 3F and A11 recognize different oligomer conformations (Figure 2, panel B). Additionally, the affinity between 10 µM Aβos and the antibody 3F obtained by incubation for 0-4 days showed that the 10 µM Aβos (sAβos, Aβos prepared *in vitro*) obtained by 2-day incubation had the highest affinity with the antibody 3F. Therefore, the subsequent experiments used this oligomer (sAβo*3F, the Apo*3F prepared *in vitro*) and the antibody 3F for immunoprecipitation (Figure 2, panel C).

### 2.3 Characterization of molecular weight of sApo*3F

The molecular weight of sApo*3F was detected by Western blot. The specific procedure was as follows: Aβ samples were loaded onto 15% SDS-PAGE gel electrophoresis or 3-8% Tris-Acetate gel. For Tris-Acetate gel, the sample was mixed with Novex^{™} Tris-Glycine sample buffer before loading. Electrophoresis was performed in Tris-Glycine electrophoresis buffer (containing 0.5 mM DTT, 1 mM ATP and 5 mM MgCl2) at 4 °C, 150 V for 4 h. For Western blot, proteins separated by SDS-PAGE or Tris-Acetate gel were transferred onto nitrocellulose membrane. The membrane was blocked with 5% skim milk for 1-2 h at room temperature, incubated with the detection antibody (6E10) at 4 °C overnight, and then washed three times with 0.1% TBST for 5 min each time, and reacted with the corresponding secondary antibody (HRP-labeled goat anti-mouse secondary antibody) for 1 h at room temperature. After washing three times with TBST, the membrane was covered with ECL developing solution, developed in an Amersham Imager 680 imaging system, and the protein bands were quantified using Image J software.

The SDS-PAGE experiment results showed that sApo*3F was mainly composed of oligomers with two different molecular weights, one was approximately 12 kDa and the other was greater than 180 kDa. The results of native-PAGE showed that sApo*3F was mainly composed of an oligomer with a molecular weight of approximately 500 kDa. The difference between the two electrophoresis results may be due to the influence of SDS (Figure 2, panel D).

In order to further determine the molecular weight of sAβo*3F, 500 µL sAβo*3F or 100 µL SEC Marker was loaded onto a Superdex 200 10/300 GL molecular exclusion chromatography column connected to an AKTA pure system. The column was pre-equilibrated with Tris-Gly buffer and eluted at a flow rate of 0.5 mL/min. By comparing with the Marker, the molecular weight of sApo*3F was found to be 588 kDa (Figure 2, panels E and F).

### 2.4 Characterization of molecular weight and morphology of mAβo*3F

The Apo*3F in the brain of APP/PS1 mice was extracted (mAβo*3F, the Apo*3F isolated from AD mouse brain) and its molecular weight was analyzed. Consistent with the results of sAβo*3F, mAβo*3F showed two bands in SDS-PAGE, with molecular weights of 12 kDa and greater than 180 kDa, respectively. While in native-PAGE, mAβo*3F had only one band with a molecular weight of about 500 kDa (Figure 2, panel G). SEC results showed that the molecular weight of mAβo*3F was 588 kDa (Figure 2, panel H). In addition, 10 µL of the Apo*3F was dropped on a 200-mesh copper grid and adsorbed for 20 min, then dried with filter paper, negatively stained with 2% uranyl acetate for 30s, dried with filter paper, and air-dried. The samples were examined under a transmission electron microscope (TEM, Hitachi H7700, Japan) at a magnification of 100,000× with 120 kV operating voltage. The results showed that mAβo*3F were particles with a diameter of about 10 nm (Figure 2, panel H).

### 2.5 Characterization of molecular weight of Apo*3F in CSF of AD patients

The molecular weight and distribution of the Apo*3F extracted from CSF of AD patients (hAβo*3F, the Apo*3F isolated from human AD patient CSF) were consistent with the results of sApo*3F and mAβo*3F. hAβo*3F had only one band in native-PAGE with a molecular weight of approximately 500 kDa, while in SDS-PAGE there were two bands with molecular weights of 12 kDa and greater than 180 kDa, respectively (Figure 2, panel I).

### Example 3 Neurotoxicity Detection of the Apo*3F

### 3.1 Experimental Materials

N2a cells: National Laboratory Cell Resource Sharing Service Platform (NICR);
D-Poly-lysine hydrobromide (PDL): Sigma-Aldrich
B27 Supplement (50x): Thermo Fisher Scientific
Neurobasal^{™}-A Medium: Gibco
70 µm nylon cell strainer: BD Bioscience

### 3.2 Primary neuron culture

The fetal brain of C57BL/6 (purchased from Beijing HFK Bioscience Co., Ltd.) at 14 to 15 days of gestation was removed, the blood and brain membranes were stripped in HBSS (Hank's balanced salt solution), and the separated hippocampus and cortex were cut into pieces with forceps. The broken tissue pieces were transferred to a 15 mL centrifuge tube and centrifuged at 600 rpm for 3 min. 10 mL of 10-fold diluted trypsin digestion solution (containing DNase I) was added to the precipitate and digested at 37 °C for 10 min, gently inverted several times every 5 min. After digestion, 40 mL of DMEM medium (containing 10% FBS) was used to terminate the trypsin digestion reaction, and the cell suspension was passed through a 70 µm cell sieve to remove undigested tissue pieces. The cells were collected by centrifugation at 1,200 rpm for 10 min, and the cells were resuspended in DMEM medium and plated in a 12-well plate. After 2-4 h, the medium was changed to Neurobasal medium (containing B27, 0.5 mM L-glutamine, 0.5% penicillin and streptomycin) and cultured for 7-9 days for subsequent experiments.

### 3.3 Cytotoxicity detection of sApo*3F

This experiment mainly used the MTT assay to detect the toxicity of Aβ aggregates to neurons. The specific procedure was as follows: N2a cells were cultured in DMEM medium containing 10% FBS and 1% penicillin/streptomycin. The cells in the culture dish were digested with trypsin, centrifuged, and resuspended in the medium, and then inoculated in a 96-well plate, with about 5,000 cells/100 µL medium per well. After 12 hours, the cells were treated with a series of concentration gradients of sApo*3F and sAβos, and the same volume of solvent was added to the cells as a control. After 72 hours, 25 µL 5 mg/mL MTT was added to each well. After further culture at 37 °C for 3 hours, the medium was aspirated and 150 µL DMSO was added. The absorbance at 570 nm and 630 nm was measured using an MD-M5 microplate reader. The average value of six replicate wells was used for each sample and control, and each experiment was repeated three times. Cell viability was calculated by dividing the absorbance (background corrected) of the sample well with the absorbance (background corrected) of the well added with solvent.

MTT results showed that the half-inhibitory concentration (IC50) of sApo*3F on cell viability was about 0.186 nM, while the IC50 of sAβos before immunoprecipitation was about 63.26 nM, there was a 340-fold difference between the two IC50s (Figure 3, panels A and B). In addition, adding 0.5 nM sApo*3F into N2a cells can cause 66% cell death, while sAβos and sAβ-ID at the same concentration had no substantial neurotoxic effect. When the concentration of sAβos reaches 200 nM, it can produce 70% cytotoxicity, while the neurotoxicity of sAβ-ID can only reach 32% at this concentration (Figure 3, panel C). These results showed that sAβo*3F is a highly neurotoxic oligomer.

### 3.4 Cytotoxicity detection of mAβo*3F

MTT assay showed that the IC50 of mAβo*3F for N2a cells was about 0.111 nM; the IC50 for primary neuronal cells was about 0.057 nM (Figure 3, panel D). Specifically, 0.5 nM mAβo*3F can cause 78% primary neuronal cell death, while the same concentrations of mAβ*6E10 and mAβ-ID had no substantial cytotoxicity. When the concentration was increased to 200 nM, mAβ*6E10 produced 54% neurotoxicity, while the neurotoxicity of mAβ-ID was only 20% (Figure 3, panel E).

### 3.5 Cytotoxicity detection of hAβo*3F

The IC50 of hAβo*3F on primary neurons detected by MTT was approximately 0.076 nM (Figure 3, panel F). Specifically, adding 0.2 nM hAβo*3F to primary neurons can reduce cell viability by 68%, while hAβ*6E10 and hAβ-ID had no significant effect on neuronal survival at this concentration. When the concentration increased to 40 nM, hAβ*6E10 reduced cell viability by 66%, while hAβ-ID only reduced cell viability by 40% (Figure 3, panel G).

### Example 4 Effect of the Apo*3F on cytokine expression levels in glial cells

### 4.1 Experimental Materials and Methods

### 4.1.1 Experimental Materials

BV2 cells: NICR
Reverse transcription kit: Kangwei Century
UltraSYBR Mixture (Low ROX): Kangwei Century

### 4.1.2 Experimental instruments

7500 Fast Real-time PCR Instrument: Applied Biosystems
T100 Thermal Cycler PCR instrument: BIO-RAD
NanoDrop Microvolume Spectrophotometer: Quawell

### 4.2 The Apo*3F can increase the expression level of inflammatory factors in microglia

BV2 cells were cultured in DMEM medium containing 10% FBS and 1% penicillin/streptomycin. The cells in the culture dish were digested with trypsin, centrifuged, resuspended in the medium, and inoculated in a 12-well plate, with approximately 5×10^5 cells/mL medium per well. After 12 h, BV2 cells were treated with different Aβ samples, and the same volume of solvent was added to the cells as a control. After 48 h, the expression level of intracellular proinflammatory factors was detected by fluorescence quantitative PCR (qPCR). The specific procedure was as follows: Total cell mRNA was obtained by conventional methods and the mRNA concentration was determined using a microtube photometer. Reverse transcription was performed according to the instructions of the reverse transcription kit to obtain cDNA, and then the expression level of the target gene was detected using EasyQuick RT MasterMix. The primers used were as follows:

| | |
|---|---|
| mTNF-α: | 5'-GATTATGGCTCAGGGTCCAA-3' (SEQ ID NO: 1), |
| | 5'-GCTCCAGTGAATTCGGAAAG-3' (SEQ ID NO: 2); |
| mIL-1β: | 5'-CCCAAGCAATACCCAAAGAA-3' (SEQ ID NO: 3), |
| | 5'-GCTTGTGCTCTGCTTGTGAG-3' (SEQ ID NO: 4); |
| mIL-6: | 5'-CCGGAGAGGAGACTTCACAG-3' (SEQ ID NO: 5), |
| | 5'-TTGCCATTGCACAACTCTTT-3' (SEQ ID NO: 6); |
| GAPDH: | 5'-TGAATACGGCTACAGCAACA-3' (SEQ ID NO: 7), |
| | 5'-AGGCCCCTCCTGTTATTATG-3' (SEQ ID NO: 8). |

The qPCR results showed that 0.3 nM mABo*3F, 200 nM mAβ*6E10 and 200 nM mAβ-ID can induce a significant increase in the expression level of proinflammatory factors in microglia, including TNF-α, IL-6 and IL-1β, but 0.3 nM mAβ*6E10 or 0.3 nM mAβ-ID had no substantial stimulatory effect (Figure 4, panel A).

### 4.3 Primary astrocyte culture

Take 1-2 day old C57BL/6 newborn mice, soak them in 75% alcohol for disinfection, remove the brain and soak in HBSS buffer, remove the blood and brain membrane, cut the brain into pieces with tissue scissors, put it in a 15 mL centrifuge tube, centrifuge at 600 rpm for 3 min, add 10 mL of 10-fold diluted trypsin digestion solution (containing DNase I ) to the precipitate, digest at 37 °C for 10 min, and gently invert a few times every 5 min. After digestion, use 40 mL DMEM medium (containing 10% FBS) to terminate the trypsin digestion reaction, and pass the cell suspension through a 70 µm cell sieve to remove undigested tissue pieces. Centrifuge at 1200 rpm for 10 min to collect cells, resuspend the cells in DMEM medium, and plate the cells in a T-75 culture flask and culture them with DMEM medium at 37 °C for 5-7 days. Change the medium every 2-3 days. After 7 days of cell culture, digest the cells with trypsin and plate them in a 12-well plate for use.

### 4.4 The Apo*3F can stimulate the expression of inflammatory factors in astrocytes

Primary astrocytes were treated with different Aβ samples, and qPCR was used to explore their effects on the expression levels of inflammatory factors in primary astrocytes. The specific procedure steps were the same as item 4.2 above. The primers used were as follows:
miNos: 5'-CACCTGGAACAGCACTCTCT-3' (SEQ ID NO: 9),
   5'-CTTTGTGCGAAGTGTCAGTG-3' (SEQ ID NO: 10);
mTNF-α: SEQ ID NOs: 1 and 2;
mIL-1β: SEQ ID NOs: 3 and 4;
mIL-6: SEQ ID NOs: 5 and 6;
GAPDH: SEQ ID NOs: 7 and 8.

The results showed that 0.3 nM mABo*3F, 200 nM mAβ*6E10 and 200 nM mAβ-ID can significantly increase the expression level of proinflammatory cytokines in primary astrocytes, including TNF-α, IL-6, IL-1β and iNos, but 0.3 nM mAβ*6E10 or 0.3 nM mAβ-ID had no significant effect on the expression level of inflammatory factors in astrocytes (Figure 4, panel B).

### 4.5 The Apo*3F disrupts astrocyte-mediated synaptogenesis.

Astrocytes can induce synapse formation by secreting synaptic factors such as TSP1 and Gpc4/6. mAβo*3F was added to primary astrocytes, and the changes in the expression level of various synaptic factors in astrocytes were detected by qPCR. The primers used were as follows:

| | |
|---|---|
| mGpc4: | 5'-CTGGAGGGTCCTTTCAACATT-3' (SEQ ID NO: 11), |
| | 5'-GACATCAGTAACCAGTCGGTC-3' (SEQ ID NO: 12); |
| mGpc6: | 5'-TAGTCCTGTATTGGCAGCCAC-3' (SEQ ID NO: 13), |
| | 5'-GGCTAATGTCTATAGCAGGGAA-3' (SEQ ID NO: 14); |
| mTSP1: | 5'-GGTAGCTGGAAATGTGGTGCGT-3' (SEQ ID NO: 15), |
| | 5'-GCACCGATGTTCTCCGTTGTGA-3' (SEQ ID NO: 16); |
| GAPDH: | SEQ ID NOs: 7 and 8. |

The results showed treating cells with 0.3 nM mAβo*3F and 200 nM mAβ*6E10 can significantly reduce the expression level of TSP1 and Gpc4/6, while 0.3 nM mAβ*6E10 or 0.3 nM mAβ-ID had no significant effect. Only when the concentration of mAβ-ID was increased to 200 nM, the expression level of Gpc4 in astrocytes was reduced (Figure 4, panel C).

### Example 5 Effects of the Apo*3F on mouse cognition and damage to brain neurons 5.1 Experimental Materials and Methods

### 5.1.1 Experimental Materials

Rapid Golgi Staining Kit: FD NeuroTechnologies
Nissl staining kit: Beijing Solarbio Science & Technology Co.,Ltd.

### 5.1.2 Experimental instruments

Brain stereotaxic injection system: Chinese Academy of Medical Sciences
Y-maze equipment: Chinese Academy of Medical Sciences
Novel object recognition equipment: Chinese Academy of Medical Sciences

### 5.2 Experimental Animals

Three-month-old C57BL/6 mice. All mice were housed in a clean room at 22 ± 2 °C and 45% ± 10% humidity, with free access to food and water, and a 12-h light and 12-h dark cycle. All animal experiments were performed in accordance with the Guidelines for the Care and Use of Laboratory Animals in Public Health Services of China, and experiments involving mice were approved by the Animal Care and Use Committee of Tsinghua University.

### 5.3 Stereotaxic Brain Injection

Three-month-old C57BL/6 mice were randomly divided into 6 groups, 6-8 mice per group, as follows: group injected with 2.5 nM (45.5 pg) mAβo*3F, group injected with 2.5 nM (45.5 pg) mAβ*6E10, group injected with 2.5 nM (45.5 pg) mAβ-ID, group injected with 600 nM (10.9 ng) mAβ*6E10, group injected with 600 nM (10.9 ng) mAβ-ID, and control group injected with Tris-Gly solvent. Mice were deeply anesthetized with a mixture of ketamine (100 mg/kg) and xylazine (10 mg/kg) and fixed on a stereotaxic injection platform. The skin of the mouse brain was cut open along the midline to expose the skull. The injection site was located using a stereotaxic instrument: starting from the bregma, AP = -1.7 mm, ML = ±1.0 mm, and DV = -1.5 mm. After opening the skull with a cranial drill along the positioning point, bilateral injections were performed, with 4 µL injected into each hemisphere at an injection rate of 0.4 mL/min. After the injection, the needle was retained for 3 min to allow the sample to be completely absorbed. The surgical site was washed with sterile saline and the incision was sutured. The status of the mice was monitored and postoperative care was provided. The behavior and cognitive ability of the mice were tested 24 h after the stereotaxic injection, and the mice were dissected for physiological and biochemical analysis.

### 5.4 The Apo*3F severely impairs the memory ability of mice

The novel object recognition test and Y-maze test were used to detect changes in the cognitive abilities of mice.

### 5.4.1 Novel Object Recognition Test

Novel object recognition test was designed based on the spontaneous tendency of mice to show more interactions with new and unfamiliar objects. The experiment was mainly divided into three stages:
(1) adaptation stage: prepare a white box with a size of 40 cm wide, 40 cm deep, and 40 cm high. Allow each mouse to freely explore the box for 5 min without any objects in it. One hour later, inject the mice with Aβ samples.
(2) training stage: 24 h later, two identical objects were placed in the box, and each mouse was placed in the same box as in the first phase, and allowed to explore freely for 5 min. The number of times the mouse sniffed and touched each object was recorded;
(3) detection stage: after 6 hours of training, the object on the right was replaced with a new object with different material, color, and shape. The mice were allowed to explore freely for another 5 minutes. The number of times the mice sniffed and touched the two objects was recorded to test the memory ability of the mice. The cognitive index was determined by the following calculation formula: (number of times the new object was touched - number of times the old object was touched) / (number of times the new object was touched + number of times the old object was touched).

The results showed that compared with the control group, the cognitive index of mice treated with 2.5 nM mAβo*3F and 600 nM mAβ*6E10 for new objects was significantly decreased, while the cognitive index of mice in other Aβ-treated groups was not significantly different from that of the control group, indicating that the Apo*3F significantly reduced the memory ability of mice (Figure 5A).

### 5.4.2 Y-maze test

After the novel object recognition test, the spatial memory ability of mice was tested by the Y-maze test. The Y-maze consists of three identical arms with a 120-degree angle between each arm. The size of each arm is 8 cm x 30 cm x 15 cm (width x length x height). The experiment was mainly divided into two stages:
(1) training stage: during the training stage, the three arms were randomly named as A, B, and C. Arms A and B were open throughout the experimental phase, while arm C was a new arm and was closed during the training stage. After being placed in arm A, each mouse was allowed to freely explore arms A and B for 10 min.
(2) after 1 h of training, the C arm was opened and the mice were placed in the A arm in order again. The mice were allowed to explore the A, B, and C arms for 5 min, and the time they stayed in the three arms was analyzed. All the experimental processes were recorded by a camera installed above the Y maze.

The results showed that the duration of mice treated with 2.5 nM mAβo*3F and 600 nM mAβ*6E10 in the new arm was significantly reduced compared with the control mice. However, there was no significant difference between the mice injected with 2.5 nM mAβ*6E10, 2.5 nM mAβ*ID and 600 nM mAβ*ID and the control group. This indicates that mAβo*3F, but not mAβ*6E10 or mAβ-ID, can cause severe memory impairment in mice at low concentrations. For mAβ*6E10, only when the concentration is increased to 600 nM can mice show a similar degree of memory impairment (Figure 5, panel B).

### 5.5 The Apo*3F significantly reduces the density of dendritic spines in mouse neurons

The mouse brain samples were stained using the FD Fast Golgi Staining Kit. 24 h before dissecting the mice, the A and B solutions were mixed in the kit in a 1:1 ratio and stored in the dark. After the mice were killed, the brain was taken directly without perfusion and divided into left and right hemispheres along the midline. The blood on the surface of the left hemisphere was washed off with PBS, and the solution on the surface of the tissue was soaked off with filter paper, and then the tissue was placed in the pre-prepared A+B solution. The solution was changed once after 24 h and placed at room temperature in the dark for 2 weeks. During the immersion period, the tissue was gently turned upside down twice a week to obtain the best results. After 2 weeks, the tissue was transferred to C solution, the solution was changed once after 24 h, and placed at room temperature in the dark for 5 days. Then, a freezing microtome was used to obtain 100 µm thick frozen sections. After the sections were dried naturally at room temperature, staining began. First, the sections were washed twice with Milli-Q water for 4 min each time, then the staining working solution was prepared, the sections were placed in the staining working solution for 15 min, and washed twice with Milli-Q water for 5 min each time. After staining, the sections were dehydrated with 50%, 75%, 95% and 100% ethanol, and each concentration gradient was processed for 4 min. The sections were then placed in xylene and sealed with neutral resin for observation. The dendrites and dendritic spines of neurons in the CA1 region of the hippocampus were observed using the 100× lens objective of an Olympus inverted microscope. The density of dendritic spines was determined by Image J software analysis (dendritic density = number of dendritic spines/dendritic length).

By calculating the density of dendritic spines, it was found that compared with the control group, the density of dendritic spines in neurons in the CA1 region of the mouse brain treated with 2.5 nM mAβo*3F and 600 nM mAβ*6E10 was significantly decreased, but 2.5 nM mAβ*6E10, 2.5 nM mAβ-ID or 600 nM mAβ-ID had no significant effect on the density of dendritic spines in mouse brain neurons (Figure 5, panels C and D).

### 5.5 The Apo*3F significantly reduces the number of neurons in mouse hippocampus

### 5.5.1 Paraffin embedding and sectioning

After behavioral experiments, the mice were deeply anesthetized with sodium pentobarbital (50 mg/kg) and a cardiac perfusion was performed with ice-cold PBS containing heparin (10 U/mL), then the mice were killed and dissected. After the brain of the mouse was removed, it was divided into the left and right hemispheres along the midline. The left hemisphere was fixed in 4% paraformaldehyde for 48 hours and then dehydrated with ethanol gradient: 50%, 70%, 80%, and 90% ethanol for 1 hour each. 100% ethanol for 1 hour, repeated once, and then 50% xylene for 30 minutes, repeated once. After dehydration, the tissue was immersed in paraffin for 4 hours and embedded. A paraffin tissue slicer was used to obtain 5 µm tissue sections.

### 5.5.1 Nissl staining

After heating at 60°C for 30 min, 5 µm paraffin sections were dewaxed by soaking in 100% xylene, 50% xylene, and 100% alcohol for 10 min each, and 75% ethanol, 50% ethanol, 30% ethanol, and deionized water for 5 min each. Then, according to the Nissl staining instructions, cresyl violet solution was drop added to the sections, heated at 56 °C for 1 h, differentiated at room temperature for 1-3 min until the background was almost colorless, decolored with gradient alcohol (70%, 95%, and 100%; 20 seconds each), and soaked in xylene. After sealing with neutral resin, data were collected and analyzed under microscope. The results showed that 2.5 nM mAβo*3F and 600 nM mAβ*6E10 caused a significant decrease in the number of neurons in the CA1 and DG regions of the mouse brain, while 2.5 nM mAβ*6E10, 2.5 nM mAβ-ID or 600 nM mAβ-ID had no significant effect on the number of neurons (Figure 5, panels E, F, G).

### Example 6 The Apo*3F activates glial cells in the mouse brain to produce neuroinflammation

### 6.1 Experimental Materials and Methods

### 6.1.1 Experimental Materials

Anti-GFAP antibody: Cell Signaling Technology, #3670
Anti-Iba-1 antibody: GeneTex, #GTX101495
BCA kit: Thermo Fisher Scientific
RIPA strong lysis buffer: Beijing Solarbio Science & Technology Co.,Ltd.
Protease inhibitors: Millipore
Phosphatase inhibitor (100x): Beijing Solarbio Science & Technology Co.,Ltd.
Mouse IL-1β Detection Kit: Biolegend
Mouse IL-6 Detection Kit: Biolegend

### 6.1.2 Experimental instruments

MD-M5 microplate reader: Molecular Devices, USA
Tissue Lyser II Tissue homogenizer: Qiagen

### 6.1.3 Immunohistochemistry

The dewaxed 5 µm paraffin sections were immersed in citric acid antigen retrieval solution (3 g trisodium citrate, and 0.4 g citric acid dissolved in deionized water, made up to 1 L), boiled in a water bath for 15 min, and cooled naturally at room temperature. After antigen retrieval, the sections were washed 3 times with PBS for 5 min each time, and then fixed with 80% (vol/vol) methanol containing 0.3% H₂O₂ to remove endogenous catalase. Then, the sections were washed 3 times with PBS for 5 min each time, placed in 0.3% Triton X-100 penetration solution, penetrated at room temperature for 20 min, and washed 3 times with PBS for 5 min each time. After the sections were blocked with 10% donkey serum at room temperature for 1 h, the corresponding primary antibody was added and incubated overnight at 4 °C. The next day, the sections were washed 3 times with PBS for 5 min each time, and the corresponding secondary antibody was added and incubated at room temperature for 1 h, and then washed 3 times with PBS for development and observation.

### 6.2 The Apo*3F can activate microglia and astrocytes

By staining the microglial marker Iba-1 in the hippocampus, it was found that 2.5 nM mAβo*3F and 600 nM mAβ*6E10 can significantly activate microglia in the DG and CA1 regions of hippocampus and increase the expression level of Iba-1 in microglia, but 2.5 nM mAβ*6E10 or mAβ-ID treatment had no significant effect on mice (Figure 6, panels A, C, and D). Moreover, 2.5 nM mAβo*3F and 600 nM mAβ*6E10 can significantly induce microglia in the DG region to enter an ameba-like state with enlarged cell bodies and fewer branches, which represents an over-activated microglia state (Figure 6, panel A). By staining the astrocyte marker GFAP in the mouse hippocampus, it was found that the injection of 2.5 nM mAβo*3F and 600 nM mAβ*6E10 into the mouse brain activated astrocytes in the DG and CA1 regions of hippocampus and increased the expression level of GFAP in hippocampus (Figure 6, panels B, E, and F).

### 6.3 The Apo*3F increases the level of inflammatory factors in the hippocampus of mice

According to the manufacturer's instructions, IL-6 and IL-1β ELISA kits were used to detect the level of inflammatory factors (IL-6 and IL-1β) in the mouse brain. Briefly, the brain homogenate was diluted and added to the ELISA plate, which was pre-coated with the corresponding capture antibody, and then the corresponding detection antibody and secondary antibody were added. TMB was used as a substrate and the absorbance was measured at 450 nm using an MD-M5 microplate reader. The results showed that the level of IL-1β and IL-6 in the hippocampus were significantly increased in mice injected with 2.5 nM mABo*3F, 600 nM mAβ*6E10, and 600 nM mAβ-ID, while the level of IL-1β and IL-6 in the brain of mice injected with 2.5 nM mAβ*6E10 or 2.5 nM mAβ-ID was not significantly different from those in the control group (Figure 6, panels G and H).

### Example 7 Detection of the Apo*3F levels in CSF and plasma of AD patients (MSD method)

### 7.1 Experimental Materials and Methods

### 7.1.1 Experimental Materials

Aβ multifactor detection kit (4G8): Meso Scale Diagnostics

### 7.1.2 Experimental Instruments

MSD-S600 Electrochemiluminescence Detector: Meso Scale Diagnostics, USA

### 7.2 Detection of the Apo*3F levels in plasma and CSF of AD patients

To evaluate the relevance of Aβos specifically recognized by the antibody 3F (Aβo*3F) and the pathogenesis of AD patients, the level of Aβ42 and Aβ40 contained in Apo*3F isolated from CSF and plasma of AD patients was tested.

The human plasma and CSF samples used in this study were approved by the Ethics Review Committee of the First Affiliated Hospital of Zhengzhou University, and all the subjects have signed written informed consent before participating in the study. The basic information of the subject samples used in this study was as follows: plasma samples were collected from 20 patients with mild cognitive impairment (MCI) with an average age of 73.3 years (age range 57-84 years), 20 AD patients with an average age of 69.4 years (age range 52-85 years), and 20 healthy people with an average age of 70.9 years (age range 65-78 years). In addition, CSF samples were collected from 7 AD patients with an average age of 68 years (age range 59-77 years) and 7 non-demented subjects with an average age of 66.3 years (age range 48-79 years).

To extract the Apo*3F from plasma and CSF of AD patients and healthy elderly people, human plasma or CSF samples were first added to 100 µL Protein A/G-agarose gel and incubated at 4 °C for 1 h to remove endogenous IgG. The samples were then incubated with Protein A magnetic beads cross-linked with the antibody 3F at room temperature for 2 h. The next day, the beads were washed three times with 0.1% PBST, eluted twice with 20 mM glycine (pH 2.0) for 3 min, and the eluent was neutralized to pH 7 with 1 M Tris. The concentration of the Apo*3F extracted by immunoprecipitation was detected using the MSD Aβ multifactor detection kit (4G8). The results showed that compared with healthy elderly people, the level of Aβ42 and Aβ40 contained in the Apo*3F in the CSF and plasma of AD patients was significantly increased (Figure 7). In addition, the level of Aβ42 and Aβ40 contained in the Apo*3F in the plasma of MCI patients was also significantly higher than those in the control group, and both gradually increased with the development of AD pathology (Figure 7, panels C and D). These results showed that the level of the Apo*3F in the CSF and plasma of AD patients and AD-derived MCI patients was significantly higher than those in healthy elderly people, and was closely related to the development trend of AD pathology, indicating that the Apo*3F can be used as a biomarker for AD diagnosis, especially for early diagnosis of AD. The statistical method of one-way ANOVA with Tukey's multiple comparison test in GraphPad Prism 8 software was used for statistics, with 95% confidence interval, *represents P < 0.05, indicating a significant statistical difference; ** represents P < 0.01, indicating a very significant statistical difference; *** represents P < 0.001, indicating an extremely significant statistical difference; **** represents P < 0.0001, indicating an extremely significant statistical difference.

### Example 8 Detection of the Apo*3F level in plasma of AD patients and MCI patients (chemiluminescence assay)

### 8.1 Experimental Materials and Methods

### 8.1.1 Experimental Materials

102 Antibody: Beijing Hotgen Biotechnology Co., Ltd.

### 8.1.2 Experimental instruments

MG60 pro chemiluminescence detector: Beijing Hotgen Biotechnology Co., Ltd., Beijing 8.2 Detection of the Apo*3F levels in plasma of AD patients

To evaluate the correlation between Aβos specifically recognized by the antibody 3F (Aβo*3F) and the pathogenesis of AD patients, the level of Aβ42 contained in the Apo*3F isolated from the plasma of AD patients was tested.

The human plasma and CSF samples used in this study were approved by the Ethics Review Committee of the First Affiliated Hospital of Zhengzhou University, and all the subjects have signed written informed consent before participating in the study. The basic information of the subject samples used in this study was as follows: plasma samples were collected from 3 AD patients with an average age of 65.6 years (age range 62-75 years), 3 MCI patients with an average age of 63.3 years (age range 62-65 years), and 3 healthy people with an average age of 63.6 years (age range 61-65 years).

In order to extract the Apo*3F from the plasma of AD patients and healthy elderly people, human plasma samples were firstly added to 200 µL Protein A-agarose gel and incubated at room temperature for 15 min to remove endogenous IgG. Then the samples were incubated with JSR magnetic beads coated with the antibody 3F at room temperature for 2 h. The next day, the magnetic beads were washed three times with 0.1% PBST, eluted with 20 mM glycine (pH 2.2) for 5 min, eluted twice, and the eluent was neutralized to pH 7 with 1 M Tris. The concentration of the Apo*3F extracted by immunoprecipitation was detected using the 3F-102 detection kit. The results showed that compared with healthy elderly people, the level of Aβ42 contained in the Apo*3F in the plasma of AD patients was significantly increased (Figure 8). In addition, the level of Aβ42 contained in the Apo*3F in the plasma of MCI patients was also significantly higher than that in the control group (Figure 8). These results showed that the level of the Apo*3F in the plasma of AD patients and MCI patients was significantly higher than those in healthy elderly people, indicating that the Apo*3F can be used as a biomarker for AD diagnosis, especially for early diagnosis of AD.

### Example 9 Diagnosis of AD by detecting the Apo*3F level in CSF or plasma

The human plasma and CSF samples in Example 7 were randomly renumbered, and given to uninformed researchers to enrich the Apo*3F in the samples using Protein A magnetic beads cross-linked with the antibody 3F according to the method of Example 7, and the level of Aβ42 and Aβ40 contained in the Apo*3F in the samples was detected using the MSD Aβ multifactor detection kit (4G8). The results showed that the amount of Aβ42 and Aβ40 contained in the Apo*3F in the samples was positively correlated with the pathology of AD. This method of detecting the level of the Apo*3F in human plasma and CSF samples can significantly distinguish AD and MCI patients from healthy samples (Figure 9).

### Example 10 Optimization of the antibodies for detecting the Apo*3F oligomers

The amino acid K at position 100 of the antibody 3F was mutated to R (the position 98 in the antibody numbering when not considering the MA residues introduced for expression as a single-chain antibody, and the variant is named as K98R mutant, and the sequence thereof is shown in SEQ ID NO: 24), the binding of K98R mutant to the Apo*3F oligomer was tested. The method used was as follows.

The above Aβ oligomers were coated onto a 96-well plate at 100 ng/well and coated overnight at 4 °C. The next day, 3% BSA was used to block the plate for 2 hours at room temperature, washed twice with PBST, and patted dry for use. K98R mutant and the antibody 3F were diluted, starting from 1 µg/ml and 2x dilution for 14 gradients. The diluted antibody solution was added to the ELISA plate coated with the above Aβ oligomers, and parallel replicate wells were made. The plate was incubated at 37 °C for 1 hour, washed 3 times with PBST, and patted dry. 1:10,000 diluted goat anti-human IgG-HRP was added to the plate, incubated at 37 °C for 45 minutes, washed 3 times with PBST, and patted dry. TMB chromogenic solution was added for color development for about 15 minutes. After the reaction was terminated with the stop solution, the OD was read at OD450 nm, and the values were analyzed and compared using graphpad and processed for graphing.

The results were shown in Figure 10. The experiment showed that the binding affinity of the K98R mutant to the Apo*3F oligomer was further improved, indicating that the higher binding ability of this mutant with the Apo*3F oligomers may be helpful for detecting the presence of the Aβo*3F oligomers in a subject more sensitively, so as to diagnose whether the subject suffers from a neurodegenerative related disease at an earlier stage. The inventors considered the reason for this result and found that when numbering according to IMGT numbering, the amino acid at position 98 is also the residue of the heavy chain CDR3 of the antibody 3F (according to IMGT numbering, the heavy chain/light chain CDR sequences of the antibody 3F are: CDR-H1: GFTFSSYA (SEQ ID NO: 25); CDR-H2: ISNLGLTT (SEQ ID NO: 26); CDR-H3: AKTTSRFDY (SEQ ID NO: 27); CDR-L1: QSISSY (SEQ ID NO: 28); CDR-L2: KAS; CDR-L3: QNSAVRPVT (SEQ ID NO: 29)). The inventors also mutated several other variant sequences to K98R, and the results were similar (data not shown).

### Equivalent solution

Although multiple embodiments of the present invention have been described and illustrated herein, a person of ordinary skill in the art would readily envision various other means and/or structures for achieving the functions described herein and/or obtaining the results and/or one or more advantages described herein, and it is believed that each such change and/or modification is within the scope of the present invention. More broadly, it will be readily understood by those skilled in the art that all the parameters, materials and settings described herein are intended to be exemplary, and that actual parameters, materials and/or settings will depend on the specific application in which the teachings of the present invention are used. Those skilled in the art would recognize or be able to determine many equivalents of the specific embodiments of the present invention described herein using only routine experiments. Therefore, it should be understood that the foregoing embodiments and examples are presented only by way of example, and within the scope of the appended claims and their equivalents, the present invention may be implemented in a manner different from that specifically described and claimed. Any combination of two or more such features, systems, objects, materials and/or methods is included within the scope of the present invention if such features, systems, objects, materials and/or methods are not mutually conflicting.

The phrase "and/or" used in the present specification and claims should be understood to mean "either or both" of the elements so combined, i.e., elements that are present in combination in some cases and not in combination in other cases. In addition to the elements specifically identified by the "and/or" clause, other elements may optionally be present, whether related or unrelated to those specifically identified elements, unless otherwise expressly indicated. Thus, as a non-limiting example, when used in combination with open language such as "comprising," a reference to "A and/or B" may refer to A without B (optionally including other elements in addition to B) in one embodiment; to B without A (optionally including elements in addition to A) in another embodiment; to both A and B (optionally including other elements) in yet another embodiment; and so on.

As used herein in the specification and claims, "or" shall be understood to have the same meaning as "and/or" as defined above. For example, when separating items in a list, "or" or "and/or" shall be understood to be inclusive, i.e., including at least one of a plurality of elements or list of elements, but also including more than one, and optionally, other unlisted items. Only when the contrary term is clearly indicated, such as "only one of" or "exactly one of", or when used in a claim, "consisting of" will refer to comprising exactly one element of a plurality of elements or lists of elements. Generally, when preceded by an exclusive term, such as "either", "one of", "only one of" or "exactly one of", the term "or" used herein should be understood only to represent an exclusive alternative (i.e., "one or the other but not both"). "Substantially consisting of" when used in a claim shall have its ordinary meaning in the field of patent law.

As used herein in the specification and claims, when referring to a list of one or more elements, the phrase "at least one" should be understood to mean at least one element selected from any one or more elements of the list of elements, but does not necessarily include at least one of each element specifically listed in the list of elements, and does not exclude any combination of elements in the list of elements. This definition also allows that any element other than the elements specifically identified in the list of elements referred to by the phrase "at least one" may optionally be present, whether related or unrelated to those specifically identified elements. Thus, as a non-limiting example, in one embodiment, "at least one of A and B" (or equivalently, "at least one of A or B", or equivalently, "at least one of A and/or B") may refer to at least one, optionally including more than one A, without B (and optionally including elements other than B); in another embodiment, to at least one, optionally including more than one B, without A (and optionally including elements other than A); in yet another embodiment, to at least one, optionally including more than one A, and at least one, optionally including more than one B (and optionally including other elements); and so on.

In the claims and the specification, all the conjunctions, such as "comprise", "include", "with", "having", "containing", "involving", "owning", etc., are understood to be open-ended, i.e., meaning including but not limited to. Only the conjunctions "consisting of" and "consisting essentially of" shall be closed or semi-closed conjunctions, respectively.

The use of ordinal terms in the claims, such as "first," "second," "third," etc. to modify claim elements, does not imply any priority, precedence, or order of one claim element relative to another claim element, or a temporal order of actions in a method, but merely serves as a label to distinguish one claim element with a certain name from another element with the same name (but for ordinal terms) to distinguish claim elements.

## Claims

1. A method for diagnosing whether a subject suffers from early and mid-late Alzheimer's disease (AD) or AD-derived mild cognitive impairment (MCI) or is at risk of developing AD, the method comprising the following steps:
a) optionally, providing a sample to be tested from a subject,
b) contacting the sample from the subject with a reagent for detecting the presence and/or level of Aβo*3F in the sample from the subject,
c) detecting the presence and/or level of the Apo*3F in the sample from the subject,
wherein the presence and/or level of the Apo*3F in the sample from the subject indicates that the subject suffers from AD or AD-derived MCI, wherein the Apo*3F is an Aβ oligomer specifically bound by the antibody 3F, and has a molecular weight of approximately 588 kDa based on molecular exclusion chromatography (SEC) analysis, and the light and heavy chain CDR sequences of the antibody 3F are as set forth in SEQ ID NOs: 17-22, respectively.

2. The method according to claim 1, wherein the subject is a patient suspected of having AD or AD-derived MCI, preferably, the subject is a human, a non-human primate, a cat or a dog.

3. The method according to claim 1 or 2, further comprising a step of clinical neuropsychological and neuroimaging assessment, preferably, the neuroimaging assessment is Aβ-PET scanning imaging and/or tau-PET.

4. The method according to any one of claims 1-3, further comprising detecting changes in the tau level in the sample from the subject, preferably, the tau level is the total tau level or the phosphorylated tau level.

5. A kit for diagnosing whether a subject suffers from AD or AD-derived MCI or is at risk of developing AD, comprising a reagent for detecting the presence and/or level of Apo*3F in a sample from the subject, wherein the Apo*3F is an Aβ oligomer specifically bound by the antibody 3F, and has a molecular weight of approximately 588 kDa based on molecular exclusion chromatography analysis, and the light and heavy chain CDR sequences of the antibody 3F are as set forth in SEQ ID NOs: 17-22, respectively.

6. The method according to any one of claims 1 to 4 or the kit according to claim 5, involving or comprising an enrichment reagent for the Apo*3F, wherein the enrichment reagent for the Apo*3F includes a binding agent specifically binding to the Apo*3F, preferably, the binding agent specifically binding to the Apo*3F is an antibody specifically binding to the Apo*3F, preferably, the antibody is a monoclonal antibody, a polyclonal antibody or an antigen-binding fragment thereof, more preferably, the antigen-binding fragment is selected from the group consisting of scFv, F(ab')2, Fab2, Fab, Fab', Fv, Fd, dAb, camelid antibody, nanobody, diabody and bispecific antibody.

7. The method or kit according to any one of claims 1 to 6, wherein the reagent for detecting the presence and/or level of the Aβo*3F comprises a first binding agent specifically binding to the Apo*3F or Aβ aggregates, preferably, the first binding agent specifically binding to the Apo*3F or Aβ aggregates is an antibody specifically binding to the Apo*3F or Aβ aggregates, preferably, the antibody is a monoclonal antibody, a polyclonal antibody or an antigen-binding fragment thereof, more preferably, the antigen-binding fragment is selected from the group consisting of scFv, F(ab')2, Fab2, Fab, Fab', Fv, Fd, dAb, camelid antibody, nanobody, diabody and bispecific antibody.

8. The method or kit according to claim 7, wherein the first binding agent is linked to a detection agent that allows for its detection.

9. The method or kit according to claim 7, wherein the reagent for detecting the presence and/or level of the Apo*3F further comprises a second binding agent specifically binding to the first binding agent, preferably, the second binding agent is an antibody specifically binding to the first binding agent, preferably, the antibody specifically binding to the first binding agent is a monoclonal antibody, a polyclonal antibody or an antigen-binding fragment thereof, more preferably, the antigen-binding fragment is selected from the group consisting of scFv, F(ab')2, Fab2, Fab, Fab', Fv, Fd, dAb, camelid antibody, nanobody, diabody and bispecific antibody, preferably, the antibody specifically binding to the first binding agent is linked to a detection agent that allows for its detection.

10. The method or kit according to claim 8 or 9, wherein the detection agent is selected from the group consisting of chemiluminescent label, electrochemiluminescent label, chromophore, fluorescent label, fluorescein-type label, umbelliferone, lissamine, cyanine, Texas Red, BODIPY FL-SE^{®} (Invitrogen) or an analog thereof, paramagnetic label, radioactive label, biotin, streptavidin/biotin, avidin/biotin, hapten, digoxin, metal complex, metal, enzyme, colloidal gold, and a combination thereof.

11. The method or kit according to any one of claims 1 to 10, wherein the detection is selected from the group consisting of chemiluminescence assay, electrochemiluminescence assay, enzyme-linked immunosorbent assay, immunofluorescence assay, immunohistochemistry assay, immunochromatography assay, radioimmunoassay, single molecule immunoassay technology (Simoa), flow cytometry, cell sorting, immunoprecipitation assay, immunodiffusion assay, dot blot assay, Western blot, protein chip, positron emission tomography and/or single photon emission computed tomography, preferably, the kit includes reagents, materials, containers and/or devices required for the detection selected from the group consisting of chemiluminescence assay, electrochemiluminescence assay, enzyme-linked immunosorbent assay, immunofluorescence assay, immunohistochemistry assay, immunochromatography assay, radioimmunoassay, single molecule immunoassay technology, flow cytometry, cell sorting, immunoprecipitation assay, immunodiffusion assay, dot blot assay, Western blot and/or protein chip; preferably, the enzyme-linked immunosorbent assay is selected from the group consisting of direct enzyme-linked immunosorbent assay, indirect enzyme-linked immunosorbent assay, direct sandwich enzyme-linked immunosorbent assay and indirect sandwich enzyme-linked immunosorbent analysis.

12. The method or kit according to any one of claims 1 to 11, wherein the sample from the subject is selected from the group consisting of cells, tissues, organs and/or body fluids of the subject, preferably, the body fluid is selected from the group consisting of whole blood, plasma, serum, cerebrospinal fluid, lymph, saliva, synovial fluid, bronchoalveolar lavage fluid, sputum, ascites, urine, amniotic fluid, peritoneal fluid, pericardial fluid, semen and/or vaginal secretions, preferably, the body fluid is selected from the group consisting of whole blood, plasma, serum and/or cerebrospinal fluid.

13. The method or kit according to any one of claims 7 to 12, wherein the first binding agent specifically binding to the Apo*3F is attached to a solid support.

14. The method or kit according to any one of claims 1 to 13, wherein the method further comprises a step of detecting a control sample, and/or the kit further comprises a control sample, wherein the control sample is from a healthy subject or a subject not suffering from AD.

15. The method or kit according to any one of claims 6 to 14, wherein the antibody specifically binding to the Apo*3F is a polyclonal antibody and/or a monoclonal antibody or an antigen-binding fragment thereof obtained by immunization against the Apo*3F, preferably, the antibody is a human antibody, a humanized antibody, a chimeric antibody, a mouse antibody, a rat antibody, a rabbit antibody, a goat antibody, a horse antibody, a sheep antibody or a non-human primate antibody.

16. The method or kit according to any one of claims 1 to 15, wherein based on the MSD electrochemiluminescence assay, the concentration of the Apo*3F in the cerebrospinal fluid of healthy population is Aβ42o*3F: 80.44 ± 20.88 pg/ml, Aβ40o*3F: 24.35 ± 5.08 pg/ml; and/or the concentration of the Apo*3F in plasma is Aβ42o*3F: 71.63 ± 36.8 pg/ml, Aβ40o*3F: 2.24 ± 0.92 pg/ml; the concentration of the Apo*3F in the plasma of population with mild cognitive impairment is Aβ42o*3F: 112.93 ± 25.02 pg/ml, Aβ40o*3F: 4.64 ± 1.43 pg/ml; the concentration of the Apo*3F in the cerebrospinal fluid of AD patient population is Aβ42o*3F: 264.8 ± 42.26 pg/ml, Aβ40o*3F: 85.74 ± 10.62 pg/ml, and/or the concentration of the Apo*3F in the plasma is Aβ42o*3F: 159.44 ± 36.8 pg/ml, Aβ40o*3F: 14.0 ± 5.59 pg/ml; or
based on the chemiluminescence assay, the concentration of Aβ42o*3F in the plasma of healthy population is 68.02 ± 39.17 pg/ml; the concentration of Aβ42o*3F in the plasma of population with mild cognitive impairment is 124.5 ± 12.57 pg/ml; and the concentration of Aβ42o*3F in the plasma of AD patient population is 205.75 ± 50.96 pg/ml.

17. The method or kit according to any one of claims 1 to 16, wherein the detection sensitivity is as low as 0.5 pg/ml based on the MSD electrochemiluminescence assay and the chemiluminescence assay.

18. The method or kit according to any one of claims 7 to 17, wherein the antibody specifically binding to the Apo*3F is a plurality of antibodies that are specific for different epitopes on the Apo*3F, such as 2, 3, 4, 5 or more antibodies that are specific for 2, 3, 4, 5 or more different epitopes on the Apo*3F, respectively.

19. The method or kit according to claim 6, wherein the step of enriching the Apo*3F from the sample is performed by immunoprecipitation using an antibody specifically binding to the Aβo*3F.
